(19) Europäisches Patentamt
European Patent Office
Office européen des brevets

(11) Publication number: **0 515 684 A1**

(12) # EUROPEAN PATENT APPLICATION published in accordance with Art. 158(3) EPC

(21) Application number: **91904623.5**

(22) Date of filing: **14.02.91**

(86) International application number: **PCT/JP91/00179**

(87) International publication number: **WO 91/11994 (22.08.91 91/19)**

(51) Int. Cl.5: **A61K 31/10**, A61K 31/12, A61K 31/135, A61K 31/155, A61K 31/16, A61K 31/185, A61K 31/19, A61K 31/415, A61K 31/42, A61K 31/695, G01N 27/26

(30) Priority: **14.02.90 JP 33545/90**

(43) Date of publication of application: **02.12.92 Bulletin 92/49**

(84) Designated Contracting States: **AT BE CH DE DK ES FR GB GR IT LI LU NL SE**

(71) Applicant: **Chugai Seiyaku Kabushiki Kaisha**
**5-1, 5-chome, Ukima Kita-ku**
**Tokyo115(JP)**

(72) Inventor: **KATO, Yasuyuki**
**2-10-13,Fuyodai**
**Mishima-shi, Shizuoka-ken(JP)**
Inventor: **TAMURA, Kunio**
**107, Nyu Izumi Haitsu, 122-2, Inari**
**Susono-shi, Shizuoka 410-11(JP)**
Inventor: **OHBA, Yasuhiro**
**206, Serizawa Haitsu, 496-1, Nameri**
**Nagaizumicho, Suntogun, Shizuoka 411(JP)**
Inventor: **KAWABE, Yoshiki**
**201, Guranderu Shuho, 440-7, Nameri**
**Nagaizumicho, Suntogun, Shizuoka 411(JP)**
Inventor: **SHINSHI, Osamu**
**B-303, Kopo Nakayama, 716-1, Nakayama**
**Gotenba-shi, Shizuoka 410(JP)**

(74) Representative: **Barth, Renate (DE)**
**Vossius & Partner Siebertstrasse 4**
**W-8000 München 80(DE)**

(54) **INHIBITOR OF DENATURED LDL FORMATION.**

(57) A pharmaceutical composition containing as the active ingredient a compound which presents low-density lipoproteins (LDL) represented by the compounds of formula (I) from being negatively charged. This composition inhibits the LDL from undergoing denaturation (oxidation) necessary for the recognition by a scavenger acceptor, and is used for treating arteriosclerosis, peptic ulcer, cancer, ischemic organ disease, inflammation and pulmonary silicosis.

EP 0 515 684 A1

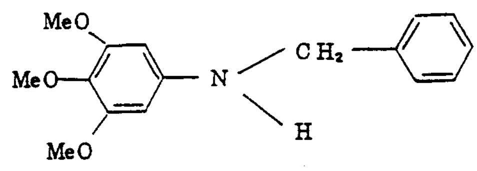

(I)

## TECHNICAL FIELD

This invention relates to a compound which suppresses the formation of denatured LDL. More particularly, it relates to a drug, which suppresses the negative charge of LDL and thus inhibits the denaturation of LDL needed in the recognition of LDL by a scavenger receptor, available as a remedy for, e.g., arteriosclerosis. The present invention further provides a method for screening a remedy for, e.g., arteriosclerosis which comprises examining the negative charge of LDL by agarose gel electrophoresis.

## BACKGROUND ART

The most common cause of ischemic cardiac diseases based on coronary lesions is arteriosclerosis. A number of clinical tests have indicated that ischemic cardiac diseases closely relate to blood cholesterol level. Thus it has been pointed out that hypercholesterolemia increases the risk of arteriosclerosis. It is believed that cholesterol transported in the blood is mostly carried by LDL (Low Density Lipoprotein) and thus LDL plays an important role in the occurrence of hypercholesterolemia. Brown et al. have clarified that defective LDL receptors, which would take up LDL (i.e., the carrier of cholesterol), are observed in cells of patients having familial hypercholesterolemia who show hereditarily high blood cholesterol levels and frequently die young from ischemic cardiac diseases and that said patients lack the ability to metabolize LDL in the blood [refer to J. Biol. Chem., 249. 5153 (1974)]. However Brown et al. have also pointed out that the metabolic pathway of cholesterol via LDL receptors does not directly relate to arteriosclerosis since those who have normal LDL receptors also suffer from arteriosclerosis. Although the metabolism of cholesterol with the LDL receptors is not effected in the case of familial hypercholesterolemia, macrophage-derived foam cells, in which cholesterol is accumulated, are observed on the arterial wall in the early stages of an arteriosclerosis lesion [refer to Med. Clin. North Am., 66, 335 (1982)]. Thus Brown et al. assumed that there might be another metabolic pathway of cholesterol which is not mediated by LDL receptors. Further, they considered that macrophages, which scarcely take up cholesterol, would take up LDL modified in vivo and thus induce the formation of foamed cells. As a result, they have determined that chemically denatured acetyl LDL (AcLDL) is taken up by macrophages and induces the formation of foamed cells.

However there is little possibility that AcLDL occurs in vivo. In order to clarify the significance of the aforesaid pathway, therefore, it is required to prove that the modification or denaturation of LDL through a reaction, which can occur in vivo in practice, induces the disordered accumulation of cholesterol by macrophages. (The AcLDL receptor is called a scavenger receptor while the accumulation of cholesterol in the cells via the aforesaid receptor is called a scavenger pathway.) With respect to the modification which might occur in vivo., it has been shown that LDL modified by endothelial cells is taken up not by LDL receptors but by macrophages via the scavenger pathway and that the modification of LDL with endothelial cells is the same as the oxidative modification of LDL with $Cu^{2+}$ [refer to Proc. Natl. Acad. Sci., USA, 78, 6499 (1981); Proc. Natl. Acad. Sci., USA, 81, 3883 (1984)]. It has been reported that the formation of TBARS (Thiobarbituric Acid Reactive Substances) in LDL, which mainly consists of cholesterol esters, phospholipdis and apo B-100, is promoted by the reaction with free amino groups of lysine in the apo B-100 lipid free radicals formed as the result of the oxidative reaction, the conversion of phosphatidylcholine into lysophosphatidylcholine and the peroxidative reaction of lipids [refer to Proc. Natl. Acd. Sci., USA, 81, 3883 (1984)]. Thus it has been found out that the oxidatively modified LDL (oxidized LDL) would induce the accumulation of cholesterol in cells via the scavenger pathway as denatured LDL capable of occurring in vivo. There have been several reports relating to the possibility of the existence of the oxidized LDL in vivo [refer to Science, 241, 215 (1988) etc.]. Furthermore, a human scavenger receptor gene was recently cloned and thus the facts of the scavenger receptor have been clarified [refer to Proc. Natl. Acad., Sci., USA, 87, 9133 (1990)].

## DISCLOSURE OF THE INVENTION

The present inventors have studied drugs capable of suppressing the formation of denatured (oxidized) LDL and considered that a substance capable of suppressing the negative charge of LDL would suppress the denaturation of LDL required in the recognition of LDL by scavenger receptors. Further, they have found out that a compound having the aforesaid properties is available as a remedy for arteriosclerosis. The aforesaid effect of suppressing the negative charge of LDL may be easily confirmed by agarose gel electrophoresis.

As will be shown by the Test Examples given hereinbelow, the present inventors have found out compounds capable of suppressing a substantial change in charge of LDL caused by the oxidative

modification with $Cu^{2+}$ by using agarose gel electrophoresis. They have furthermore proved, by degradation assay with the use of mouse peritoneal macrophages, that the aforesaid compounds suppress the formation of oxidized LDL with $Cu^{2+}$ and thus inhibit the uptake of said LDL into cells via the scavenger pathway. They have furthermore found out that these compounds suppress the TBARS level increased by the oxidation with $Cu^{2+}$ and that the effect of suppressing the TBARS level correlates to the effect of suppressing the mobility in agarose gel electrophoresis. The present invention relates to the use of a compound capable of suppressing the negative charge of LDL as a drug, in particular, a remedy for arteriosclerosis. The change in the negative charge of LDL can be confirmed by agarose gel electorophoresis or by examining the effect of suppressing the TBARS level. The compounds having the aforesaid effects are further available as a treatment for peptic ulcers, cancer, ischemic organopathy, inflammation and pulmonary diseases caused by, for example, silicon dust, in addition to arteriosclerosis.

Now and example of the compound of the present invention and a method for producing the same will be illustrated.

1) A compound represented by the following general formula (I):

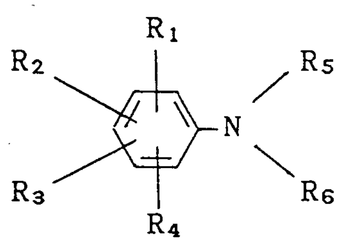

(I)

wherein $R_1$, $R_2$, $R_3$ and $R_4$ are each selected from a group consisting of a hydrogen atom, a hydroxy group, an optionally branched alkyl group having 1 to 5 carbon atoms, an alkoxy group having 1 to 5 carbon atoms, a methylthio group, a trimethylsilyloxy group, a methylenedioxy group, a halogen atom and a phenyl group;

$R_5$ is selected from a group consisting of a group represented by the following general formula (I)-1:

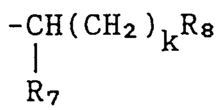

(I) - 1

wherein $R_7$ is selected from a group consisting of a hydrogen atom, an alkyl group having 1 to 5 carbon atoms, an alkenyl group having 1 to 5 carbon atoms, a phenyl group and a cyano group;

k is an integer of from 0 to 8; and

$R_8$ is selected from a group consisting of an optionally branched alkyl group having 1 to 20 carbon atoms, an optionally branched alkenyl group having 1 to 20 carbon atoms optionally substituted with a phenyl group, an optionally substituted phenyl group, an optionally substituted heterocyclic group, a cycloalkyl group having 3 to 8 carbon atoms, a naphthyl group, an adamantyl group, a tosyloxy group, a hydroxy group and a group represented by the following general formula:

$CO_2R_9$

wherein $R_9$ is selected from a group consisting of a hydrogen atom and an alkyl group having 1 to 5 carbon atoms;

a group represented by the following general formula (I)-2:

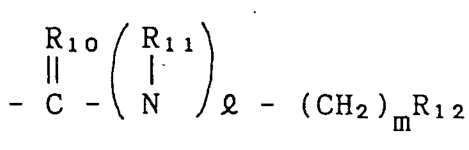

(I) - 2

wherein $R_{10}$ is selected from a group consisting of O, S and NCN;

$R_{11}$ represents a hydrogen atom or an optionally branched alkenyl group having 1 to 20 carbon

atoms;

ℓ is an integer of 0 or 1;

m is an integer of from 0 to 10; and

$R_{12}$ is selected from a group consisting of an optionally branched alkyl group having 1 to 10 carbon atoms, an alkenyl group having 1 to 5 carbon atoms optionally substituted with a phenyl group, an alkoxy group having 1 to 5 carbon atoms, an optionally substituted phenyl group, a trifluoromethyl group, an alkylthio group having 1 to 20 carbon atoms, a halogen atom, a pyridyl group and a chloromethyl group; a decalyl group, a tetralyl group, an adamantyl group, a tosyl group and a chromanyl group; and

$R_6$ is selected from a group consisting of a hydrogen atom, an alkyl group having 1 to 20 carbon atoms, a group represented by the following general formula (I)-3:

$-(CH_2)_n-R_{13}$  (I) - 3

wherein n is an integer of from 1 to 6; and $R_{13}$ is selected from a group consisting of a hydroxy group, an optionally substituted phenyl group, a cyclohexyl group and an optionally substituted carboxyl group;

a group represented by the following general foumula (I)-4:

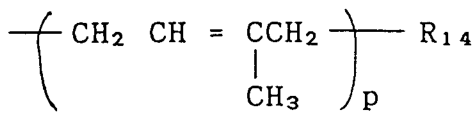

(I) - 4

wherein p is an integer of from 1 to 3; and

$R_{14}$ represents a hydrogen atom or an optionally branched alkyl group having 1 to 20 carbon atoms; and a group represented by the following general formula (I)-5:

$-CH_2CH=CHR_{15}$  (I) - 5

wherein $R_{15}$ represents a hydrogen atom or a phenyl group; or

$R_6$ may form each of the groups represented by the following general formulae together with $R_5$:

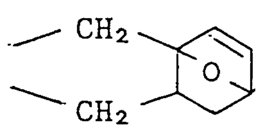

,

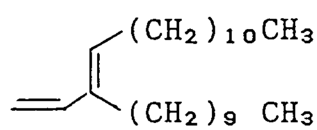

,

and

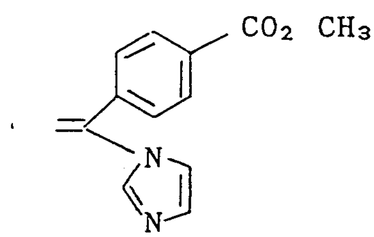

;

or a salt thereof.

2) A compound represented by the following general formula (II):

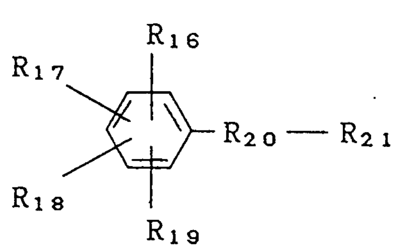

(II)

wherein $R_{16}$, $R_{17}$, $R_{18}$ and $R_{19}$ are each selected from a group consisting of a hydrogen atom, a hydroxy group, an optionally branched alkyl group having 1 to 5 carbon atoms and an alkoxy group having 1 to 5 carbon atoms;

$R_{20}$ is selected from a group consisting of O, S, a methylene group and a phenylene group; and

$R_{21}$ a group represented by the following general formula (II)-1:

$-NHR_{22}$ (II) - 1

wherein $R_{22}$ is selected from a group consisting of an optionally branched alkyl group having 1 to 15 carbon atoms, an optionally branched alkenyl group having 1 to 15 carbon atoms and a benzyl group;

and an optionally branched alkenyl group having 1 to 20 carbon atoms;

or a salt thereof.

3) A compound-represented by the following general formula (III):

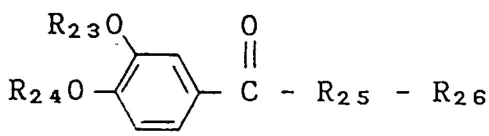

(III)

wherein $R_{23}$ and $R_{24}$ represent each a hydrogen atom or an acetyl group;

$R_{25}$ represents -NH- or a group represented by the following general formula:

$(CH2)_q$

wherein q is an integer of from 0 to 3;

$R_{26}$ is selected from a group consisting of a group represented by the following general formula (III)-1:

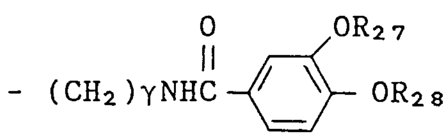

(III) - 1

wherein r is an integer of from 1 to 15; and

$R_{27}$ and $R_{28}$ represent each a hydrogen atom or an acetyl group;

a group represented by the following general formula (III)-2:

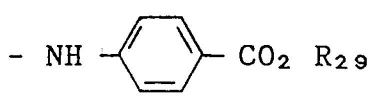

(III) - 2

wherein $R_{29}$ represents an alkyl group having 1 to 5 carbon atoms;

an optionally substituted phenyl group, an optionally substituted piperazinyl group and a pyridyl group;

or a salt thereof.

4) A compound represented by the following general formula (IV):

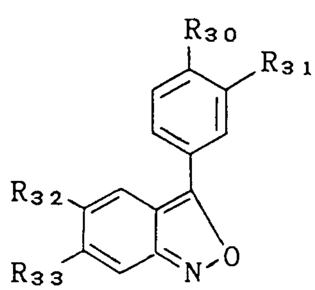

(IV)

wherein $R_{30}$ and $R_{31}$ represent each a hydrogen atom or a hydroxy group; and

$R_{32}$ and $R_{33}$ represent each a hydrogen atom or a halogen atom;

or a salt thereof.

5) A compound represented by the following general formula (V):

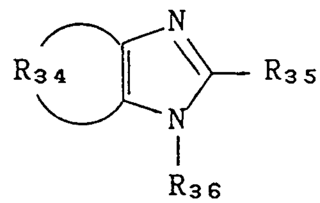

(V)

wherein $R_{34}$ forms a 5- to 7-membered ring which is optionally substituted and may contain 1 or 2 nitrogen atoms; and

$R_{35}$ and $R_{36}$ are each selected from a group consisting of a hydrogen atom, an optionally branched alkyl group having 1 to 20 carbon atoms and an optionally substituted alkenyl group having 1 to 20 carbon atoms;

or a salt thereof.

6) a compound represented by the following general formula (VI):

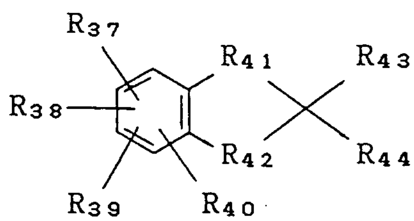

(VI)

wherein $R_{37}$, $R_{38}$, $R_{39}$ and $R_{40}$ are each selected from a group consisting of a hydrogen atom, a hydroxyl group and an alkoxy group having 1 to 5 carbon atoms;

$R_{41}$ is a group represented by the following general formula (VI)-1:

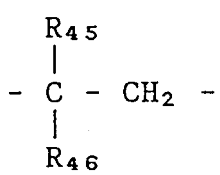

(VI) - 1

wherein $R_{45}$ and $R_{46}$ are each selected from a group consisting of a hydrogen atom, a hydroxy group and an alkyl group having 1 to 5 carbon atoms;

or each of the groups represented by the following general formulae:

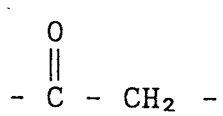 and 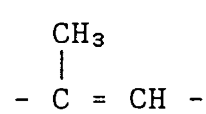 ;

$R_{42}$ is an oxygen atom or a group represented by the following general formula (VI)-2:

$-NR_{47}-$ (VI) - 2

wherein $R_{47}$ is selected from a group consisting of a hydrogen atom, an alkyl group having 1 to 5 carbon atoms and a benzyl group; and

$R_{43}$ and $R_{44}$ are each selected from a group consisting of a hydrogen atom, an alkyl group having 1 to 5

carbon atoms and an optionally substituted phenyl group;

of a salt thereof.

The compound represented by the general formula (I) may be obtained by, for example, the following methods.

a) It may be generally synthesized by the following method.

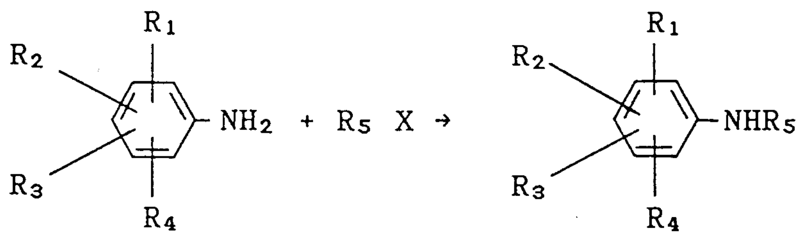


(1) (2) (3)

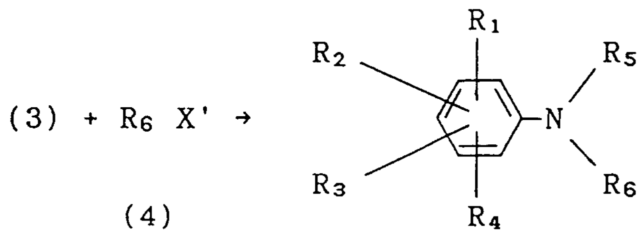


(4)

(I)

b) When $R_5$ is a $CH_2R_5$' group, the following method may be used.

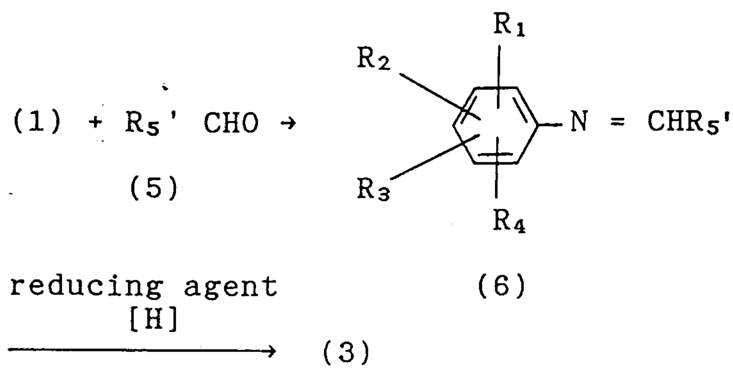


(5) (6)

c) When $R_6$ is a $CH_2R_6$' group, the following method may be used.

(3) + $R_6$' CHO ⟶ (I)
(7)

d) When $R_5$ is a $CHR_5'R_5''$ group and $R_6$ is a hydrogen atom, the following method may be used.

(5) + $R_5$" MgX" →
(8)

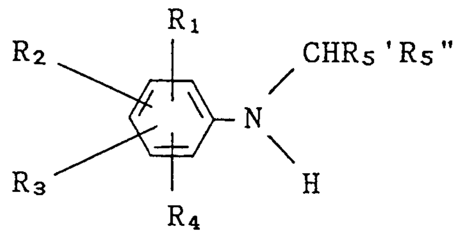

(I)

e) When $R_5$ is a $CH_2R_5$' group and $R_6$ is a hydrogen atom, the following method amy be used.

$R_5$' COCℓ
(9)
(1) →
or $R_5$' $CO_2H$,
(10)

dicyclohexylcarbodiimide (DCC)

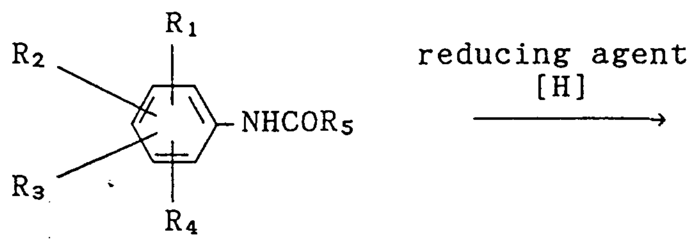


(II)

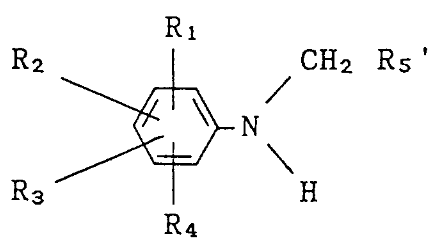

(I)

f) When the general formula (I) is represented by the following general formula (I'), the following method may be used.

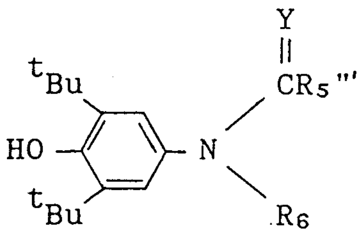

(I)'

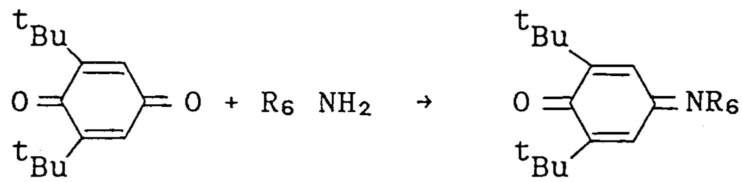

(12) (13) (14)

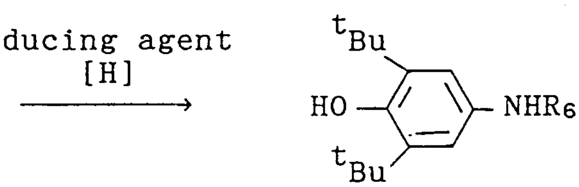


(15)

(15) + $R_5'''$ NCY → (I)'
(16)

When Y is an oxygen atom, in particular, the following method may be used.

$R_5'''$ COCℓ
(17)
(15) → (I)'
or, $(R_5''' CO)_2O$
(18)

wherein $R_1$, $R_2$, $R_3$, $R_4$, $R_5$ and $R_6$ are as defined above;

X, X', and X'' are the same or different and each represents a leaving group; and

Y represents O or S.

The reaction for obtaining the compound of the general formula (3) from the compound of the general formula (I) and the compound of the general formula (2) and the reaction for obtaining the compound of the general formula (I) from the compound of the general formula (3) and the compound of the general formula (4) may be performed in a solvent such as N,N-dimethylformamide in the presence of, for example, 1,8-diazabicyclo[5,4,0]-7-undecene (DBU) or sodium hydride under stirring at 0 ° C.

The reaction for obtaining the compound of the general formula (6) from the compound of the general formula (I) and the compound of the general formula (5) may be performed in a solvent such as benzene by heating under reflux. The reaction for obtaining the compound of the general formula (3) from the compound of the general formula (6) may be performed in a solvent such as methanol in the presence of, for example, sodium borohydride under stirring at room temperature.

The reaction for obtaining the compound of the general formula (I) from the compound of the general formula (3) and the compound of the general formula (7) may be performed in a solvent such as

acetonitrile in the presence of, for example, sodium cyano borohydride or acetic acid under stirring at room temperature.

The reaction for obtaining the compound of the general formula (I) from the compound of the general formula (5) and the compound of the general formula (8) may be performed in a solvent such as tetrahydrofuran by heating under reflux.

The reaction for obtaining the compound of the general formula (11) from the compound of the general formula (1) and the compound of the general formula (9) may be performed in the presence of, for example, triethylamine in a solvent such as chloroform under stirring. The reaction for obtaining the compound of the general formula (I) from the compound of the general formula (11) may be performed in the presence of, for example, lithium aluminum hydride in a solvent such as tetrahydrofuran by heating under reflux.

The reaction for obtaining the compound of the general formula (14) from the compound of the general formula (12) and the compound of the general formula (13) may be performed by suspending in, for example, benzene in the presence of, for example, p-toluenesulfonic acid and heating under reflux. The reaction for obtaining the compound of the general formula (15) from the compound of the general formula (14) may be performed by suspending in, for example, ethanol in the presence of, for example, sodium borohydride and stirring at room temperature.

The reaction for obtaining the compound of the general formula (I)' from the compound of the general formula (15) and the compound of the general formula (18) may be performed by stirring in a solvent such as pyridine at room temperature.

Examples of the leaving groups represented by X, X' and X'' in the formula include halogen atoms such as chlorine, bromine and iodine atoms.

The compound represented by the general formula (II) may be obtained by, for example, the following method.

g) When $R_{20}$ is a methylene group and $R_{21}$ is $NHR_{22}$, it may be synthesized by the following method.

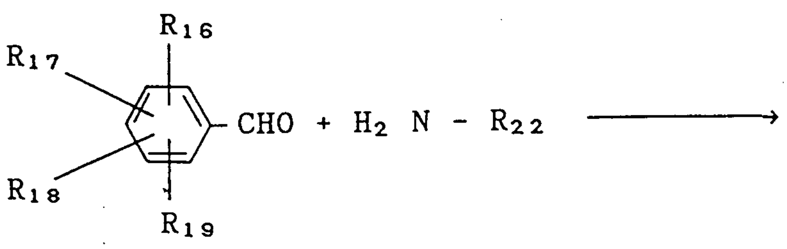

(19)

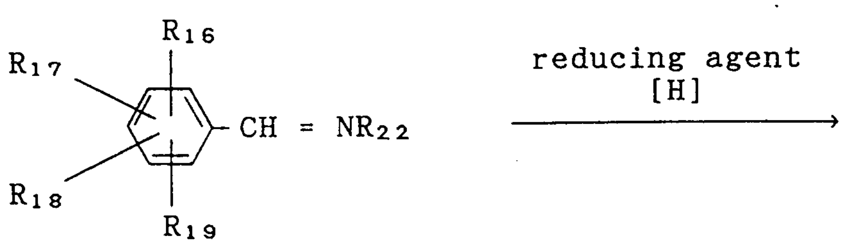


(21)

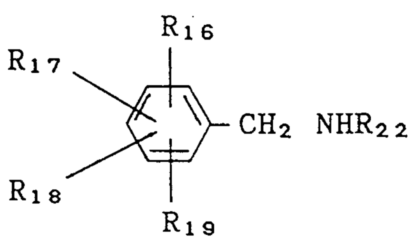

(II)

h) When $R_{21}$ is $NHCH(CH_3)R_{22}'$, the following method may be used.

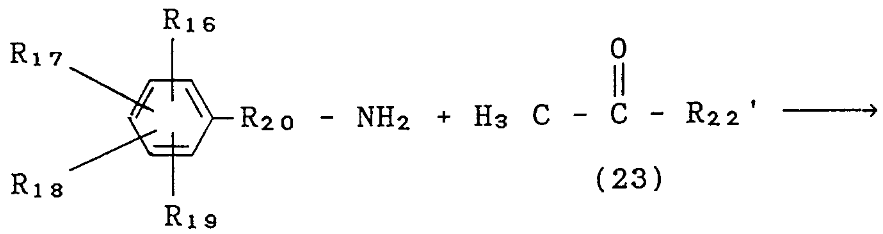

(23)

(22)

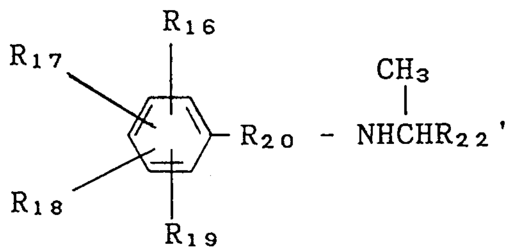

(II)

wherein $R_{16}$, $R_{17}$, $R_{18}$, $R_{19}$, $R_{20}$, $R_{21}$ and $R_{22}$ are as defined above.

The reaction for obtaining the compound of the general formula (21) from the compound of the general formula (19) and the compound of the general formula (20) may be performed in the presence of, for example, p-toluenesulfonic acid in a solvent such as benzene by heating under reflux. The reaction for obtaining the compound of the general formula (II) from the compound of the general formula (21) may be performed in the presence of, for example, sodium borohydride in a solvent such as methanol by stirring at room temperature.

The reaction for obtaining the compound of the general formula (II) from the compound of the general formula (22) and the compound of the general formula (23) may be performed in the presence of, for example, sodium borohydride cyanide, sodium sulfate anhydride, acetic acid and dry methanol under a nitrogen gas stream by stirring at room temperature.

The compound represented by the general formula (III) may be obtained by, for example, the following method.

i) When $R_{25}$ is -NH-, it may be synthesized as follows.

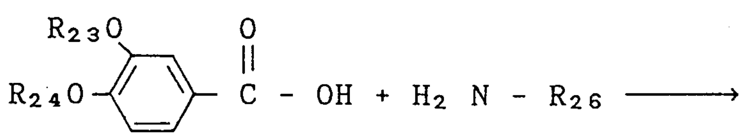

(24) (25)

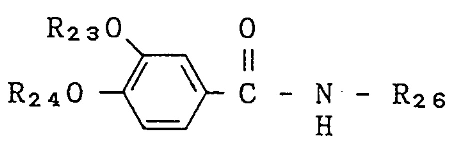

(III)

wherein $R_{23}$, $R_{24}$, $R_{25}$ and $R_{26}$ are as defined above.

The reaction for obtaining the compound of the general formula (III) from the compound of the general formula (24) and the compound of the general formula (25) may be performed as follows. First,

the compound of the general formula (24) is heated under reflux in a solvent such as chloroform in the presence of, for example, thionyl chloride to thereby give an acid chloride. Next, the compound of the general formula (25) and the acid chloride obtained above are stirred in a solvent such as chloroform in the presence of, for example, triethylamine at room temperature. Thus the compound of the general formula (III) was obtained.

The compound represented by the general formula (IV) may be obtained by, for example, the following methods.

j) It may be generally synthesized as follows.

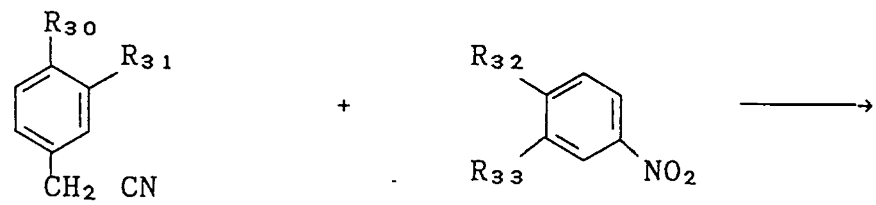

(26)

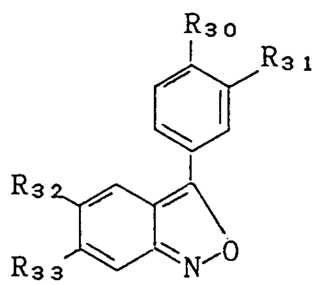

(IV)

k) When $R_{30}$ and $R_{31}$ are each OH, it may be synthesized by the following method.

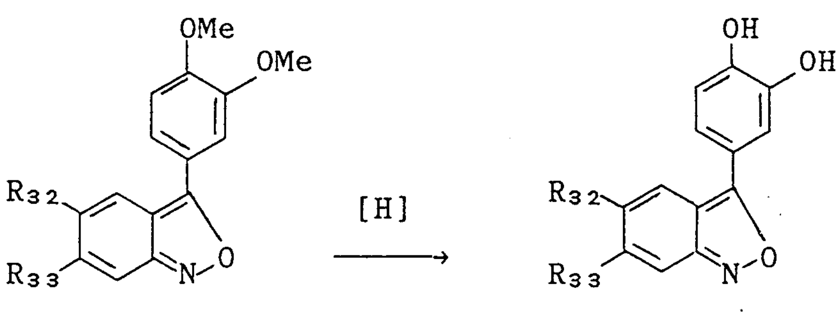


(28) (IV)

wherein $R_{30}$, $R_{31}$, $R_{32}$ and $R_{33}$ are as defined above.

The reaction for obtaining the compound of the general formula (IV) from the compound of the general formula (26) and the compound of the general formula (27) may be performed in a solvent such as methanol in the presence of, for example, potassium hydroxide by stirring at room temperature.

The reaction for obtaining the compound of the general formula (IV) from the compound of the general formula (28) may be performed by suspending the compound of the general formula (28) in, for example, hydroiodic acid and then heating under reflux.

The compound represented by the general formula (V) may be obtained by, for example, the following method.

1) It may be generally synthesized as follows.

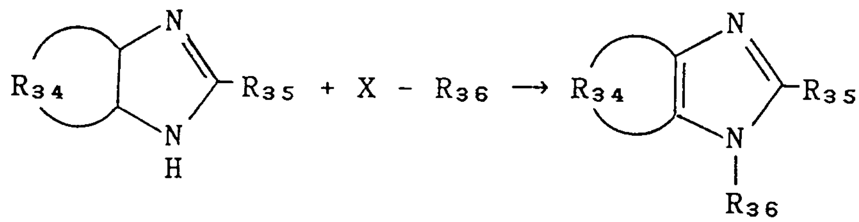

(29) (30) (V)

wherein $R_{34}$, $R_{35}$ and $R_{36}$ are as defined above; and

X represents a leaving group.

The reaction for obtaining the compound of the general formula (V) from the compound of the general formula (29) and the compound of the general formula (30) may be performed in a solvent such as dimethylformamide in the presence of, for example, DBU under stirring.

Examples of the leaving group represented by X in the formula include halogen atoms such as chlorine, bromine and iodine atoms.

The compound represented by the general formula (VI) may be obtained by, for example, the following method.

m) When the general formula (IV) corresponds to the following general formula (VI)':

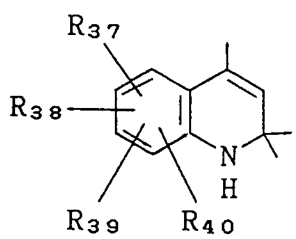

(VI)'

it may be synthesized by the following method:

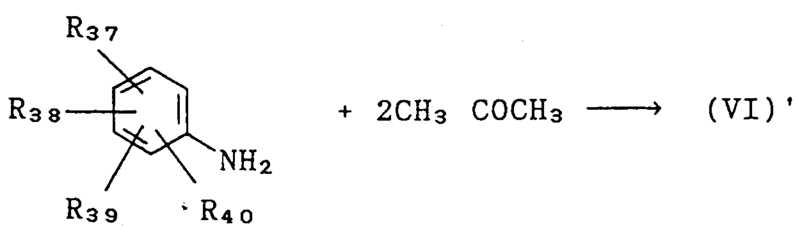


(31)

wherein $R_{37}$, $R_{38}$, $R_{39}$ and $R_{40}$ are as defined above.

The reaction for obtaining the compound of the general formula (IV)' from the compound of the general formula (31) may be performed in the presence of, for example, acetic acid by heating under reflux.

## FUNCTION

The compound of the present invention suppresses the negative charge of LDL and thus suppresses the denaturation of LDL required in the recognition of LDL by a scavenger receptor. This function may be confirmed by, for example, the examinations as shown below.

(1) The amount of thiobarbituric acid reactive substances.

(2) Effect on lipoperoxide radicals formed by autoxidation of linoleic acid.

(3) Measurement of electrophoretic mobility in agarose gel.

(4) Measurement of degradation in mouse peritoneal macrophages.

(Method)

The biological properties of the compounds as shown hereinafter were examined by the following methods.

(1) The amount of thiobarbituric acid reactive substances:

5 $\mu$M of $Cu^{2+}$ was added to rabbit LDL, prepared by the method reported by Havel et al., followed by heating. Then the antioxidative effect of each compound was examined by using the thiobarbituric acid reactive substances (TBARS) thus formed as the guidance. Table 1 shows the results.

(2) Effect on lipoperoxide radicals formed by autoxidation of linoleic acid (antioxidative effect):

The effect on lipoperoxide radicals formed by autoxidation of linoleic acid was examined by using a firely luciferin derivative (2-methyl-6-(p-methoxyphenyl)-3,7-dihydroimidazo[1,2-a]pyrazin-3-one: MCLA) as a sensitizer for the lipoperoxide radicals. 0.5 ml of an n-butanol solution containing 0.2 $\mu$M of MCLA and 10 mM of linoleic acid was introduced into a vial for luminescence analysis and the luminescence due to autoxidation was measured in a thermostat at 37° C. Table 2 shows the results.

(3) Measurement of electrophoretic mobility in agarose gel:

Rabbit or human blood collected in EDTA was centrifuges at 4° C at 3,000 rpm for 30 minutes to thereby give the plasma. To the obtained plasma, were added EDTA-$NaN_3$ (a 5% solution of pH 7.4) and a benzamidine solution (60 mg/ml) respectively in amounts of 0.8 ml and 0.5 ml per 100 ml of the plasma. Then rabbit or human LDL (1.019 < d < 1.063) was prepared by ultracentrifugation in accordance with the method of Havel et al.*. After performing the ultracentifugation again, the LDL was washed and concentrated. Then it was dialyzed against a 150 mM NaCl - 2 mM $Na_2HPO_4$ solution at 4° C and KBr was removed. The protein content was determined by Lowry method** and then the LDL was subjected to the subsequent procedure.

(Measurement of electrophoretic mobility in agarose gel)

10 $\mu$M of $Cu^{2+}$ and a specimen were added to an LDL-containing solution (3.00 $\mu$g protein/ml). After incubating at 37° C for approximately 24 hours, a portion (1 $\mu$l) thereof was applied onto an agarose gel film (Universal Film, manufactured by Corning Co.) and then subjected to electrophoresis (Agarose Gel Electrophoresis System for Lipoprotein, manufactured by Corning Co.). Thus the mobility was measured by staining lipids with Fat Red 7B. Table 3 shows the results.

(4) Measurement of degradation in mouse peritoneal macrophages:

Thioglycollate was intraperitoneally administered to a mouse. After 3 days, peritoneal macrophages were collected from the mouse and incubated in an RPMI 1640 medium containing 10% of FBS. The macrophages were used in the examination on the next day.

$^{125}$I-LDL was prepared from LDL by using $Na^{125}I$ in accordance with McFarian's method***. Free $^{125}$I was removed by passing the mixture through a PD-10 column (manufactured by Pharmacia) and dialyzing. Further, the mixture was passed through an NAP-5 column (manufactured by Pharmacia) to thereby remove EDTA. To a solution containing the $^{125}$I-LDL (50 - 100 $\mu$g protein/ml), were added 5 to 25 $\mu$M of $Cu^{2+}$ and a specimen. After incubation at 37° C for approximately 24 hours, $^{125}$I-oxidized LDL was obtained. 5 $\mu$g protein/ml of the obtained $^{125}$I-oxidized LDL was added to the mouse peritoneal macrophages (3 x $10^5$/well in a 24-well plate) and then incubated at 37° C for 5 hours. Then the $^{125}$I-tyrosine thus liberated into the medium was counted in accordance with the method reported by Goldstein et al.****. The protein of the macrophages was determined by Lowry Method** and thus the degradation per mg protein of the macrophages was determined.

In order to determine the nonspecific degradation, maleyl BSA, which is the ligand for scavenger receptors, was added to the cells in such an amount as to give a final concentration of 200 $\mu$g/ml together with the $^{125}$I-oxidized LDL in the case of each specimen. As the equation given hereinbelow shows, the effect of each specimen was calculated by subtracting the nonspecific degradation from the total degradation. Table 4 shows the results. Reference employed in the above (1) to (4): *Havel, R.J. et al., J. Clon. Invest., 34, 1345 - (1955) **Lowry, O.H. et al., J. Biol. Chem., 193, 265 - (1951) ***McFariane, A.S. et al., Nature, 182, 53 - (1958) ****Goldstein, J.L. et al., Method in Enzymology, 98, 241 - (1983).

Table 1

| Compound* | Formed TBARS (%) ** | |
|---|---|---|
| | (Compound conc. $10^{-6}$ M) | (Compound conc. $10^{-5}$ M) |
| 1 | 49 | 33 |
| 2 | 56 | 31 |
| 3 | 51 | 35 |
| 8 | 55 | 31 |
| 9 | 78 | 57 |
| 10 | 25 | 9 |
| 26 | 27 | 12 |
| 27 | 29 | 12 |
| 28 | 30 | 13 |
| 29 | 39 | 20 |
| 30 | 57 | 26 |
| 35 | 52 | 28 |
| 41 | 63 | 16 |
| 42 | 72 | 19 |
| 43 | 94 | 62 |
| 44 | 66 | 13 |
| 45 | 80 | 15 |
| 46 | 69 | 13 |
| 47 | 76 | 17 |
| 48 | 90 | 40 |
| 49 | 78 | 39 |
| 50 | 70 | 17 |
| 51 | 32 | 16 |

Table 1 (contd.)

| Compound* | Formed TBARS (%) ** (Compound conc. $10^{-6}$ M) | (Compound conc. $10^{-5}$ M) |
|---|---|---|
| 52 | 43 | 13 |
| 53 | 38 | 13 |
| 54 | 46 | 16 |
| 55 | 46 | 20 |
| 56 | 77 | 59 |
| 57 | 34 | 12 |
| 60 | 46 | 17 |
| 64 | 38 | 17 |
| 67 | 55 | 35 |
| 69 | 36 | 18 |
| 70 | 25 | 18 |
| 71 | 20 | 7 |
| 75 | 38 | 16 |
| 77 | 31 | 17 |
| 78 | 29 | 14 |
| 79 | 46 | 19 |
| 80 | 33 | 17 |
| 81 | 25 | 15 |
| 82 | 34 | 15 |
| 83 | 28 | 15 |
| 85 | 64 | 20 |
| 86 | 52 | 27 |
| 90 | 26 | 9 |

Table 1 (contd.)

| Compound* | Formed TBARS (%) ** | |
|---|---|---|
| | (Compound conc. $10^{-6}$ M) | (Compound conc. $10^{-5}$ M) |
| 94 | 87 | 63 |
| 95 | 66 | 29 |
| 96 | 67 | 43 |
| 97 | 44 | 24 |
| 98 | 79 | 43 |
| 99 | 79 | 47 |
| 100 | 81 | 13 |
| 101 | 41 | 18 |
| 102 | 27 | 15 |
| 103 | 23 | 8 |
| 104 | 31 | 23 |
| 105 | 22 | 8 |
| 106 | 16 | 8 |
| 107 | 20 | 7 |
| 108 | 21 | 8 |
| 109 | 20 | 10 |
| 110 | 16 | 7 |
| 113 | 66 | 29 |
| 114 | 94 | 93 |
| 115 | 71 | 15 |
| 116 | 61 | 33 |
| 117 | 63 | 35 |
| 118 | 56 | 37 |

Table 1 (contd.)

| Compound* | Formed TBARS (%) ** | |
|---|---|---|
| | (Compound conc. $10^{-6}$ M) | (Compound conc. $10^{-5}$ M) |
| 119 | 66 | 26 |
| 120 | 66 | 24 |
| 121 | 68 | 44 |
| 122 | 68 | 31 |
| 123 | 73 | 39 |
| 125 | 25 | 12 |
| 126 | 41 | 16 |
| 127 | 86 | 14 |
| 128 | 93 | 65 |
| 130 | 98 | 87 |
| 136 | 71 | 51 |
| 137 | 53 | 42 |
| 138 | 35 | 18 |
| 139 | 87 | 45 |
| 140 | 65 | 36 |
| 142 | 68 | 41 |
| 144 | 88 | 89 |
| 145 | 91 | 84 |
| 146 | 88 | 88 |
| 147 | 69 | 13 |
| 148 | 71 | 19 |
| 149 | 73 | 19 |
| 152 | 63 | 33 |

Table 1 (contd.)

| Compound* | Formed TBARS (%) ** | |
|---|---|---|
| | (Compound conc. $10^{-6}$ M) | (Compound conc. $10^{-5}$ M) |
| 157 | 87 | 68 |
| 158 | 94 | 47 |
| 159 | 88 | 85 |
| 167 | 95 | 96 |
| 170 | 83 | 26 |
| 172 | 12 | 4 |
| 174 | 69 | 34 |
| 176 | 65 | 29 |
| 177 | 49 | 23 |
| 178 | 90 | 83 |
| 179 | 63 | 15 |
| 180 | 64 | 17 |
| 182 | 51 | 18 |
| 183 | 91 | 47 |
| 184 | 52 | 14 |
| 185 | 30 | 7 |
| 186 | 70 | 34 |
| 188 | 61 | 10 |
| 189 | 86 | 68 |
| 190 | 83 | 32 |

Table 1 (contd.)

| Compound* | Formed TBARS (%) ** | |
|---|---|---|
| | (Compound conc. $10^{-6}$ M) | (Compound conc. $10^{-5}$ M) |
| 191 | 95 | 95 |
| 194 | 14 | 3 |
| 197 | 15 | 5 |
| 205 | 10 | 3 |
| 206 | 86 | 58 |
| 207 | 89 | 59 |
| 208 | 91 | 60 |
| 209 | 65 | 48 |
| 210 | 85 | 82 |
| 211 | 83 | 47 |
| 212 | 22 | 10 |
| 213 | 7 | 5 |
| 214 | 41 | 8 |
| Control | 100 | |

* Each compound No. corresponds to that given in Table 5.

**

$$\text{Formed TBARS} = \frac{\text{TBARS formed at the addition of specimen}}{\text{TBARS formed in solvent}} \times 100$$

Table 2

| Compound* | MCLA (%)** (Compound conc. 2 x $10^{-4}$ M) |
|---|---|
| 25 | 5 |
| 26 | 13 |
| 27 | 8 |
| 28 | 5 |
| 29 | 12 |
| 41 | 32 |
| 42 | 51 |
| 44 | 37 |
| 45 | 7 |
| 46 | 50 |
| 47 | 25 |
| 48 | 26 |
| 49 | 15 |
| 50 | 5 |
| 51 | 12 |
| 52 | 20 |
| 53 | 22 |
| 54 | 33 |
| 55 | 26 |
| 56 | 27 |
| 57 | 15 |
| 69 | 10 |
| 70 | 12 |

Table 2 (contd.)

| Compound* | MCLA (%)** (Compound conc. 2 x $10^{-4}$ M) |
|---|---|
| 71 | 23 |
| 72 | 33 |
| 74 | 14 |
| 77 | 11 |
| 78 | 9 |
| 80 | 10 |
| 81 | 11 |
| 82 | 8 |
| 84 | 5 |
| 87 | 34 |
| 93 | 37 |
| 95 | 52 |
| 96 | 48 |
| 97 | 12 |
| 98 | 8 |
| 99 | 7 |
| 100 | 8 |
| 101 | 6 |
| 102 | 10 |
| 103 | 12 |
| 104 | 14 |
| 105 | 6 |
| 106 | 9 |

Table 2 (contd.)

| Compound* | MCLA (%)** (Compound conc. 2 x $10^{-4}$ M) |
|---|---|
| 107 | 4 |
| 108 | 2 |
| 109 | 7 |
| 111 | 4 |
| 142 | 49 |
| 166 | 41 |
| 170 | 0 |
| 171 | 22 |
| 172 | 1 |
| 173 | 53 |
| 175 | 11 |
| 182 | 41 |
| 186 | 33 |
| 189 | 1 |
| 190 | 32 |
| 191 | 35 |
| 194 | 7 |
| 205 | 3 |
| 208 | 16 |
| 210 | 35 |
| 213 | 3 |
| 214 | 10 |
| Control | 56 |

*: Each compound No. corresponds to that given in Table 5.

**:

$$MCLA\ (\%) = \frac{\text{Luminescence intensity after adding specimen or solvent}}{\text{Luminescence intensity before adding specimen or solvent}} \times 100\ (\%)$$

Table 3

| Compound* | Compound conc. (x $10^{-6}$ M) | Mobility** |
|---|---|---|
| 1 | 10 | 1.17 |
| 118 | 10 | 1.17 |
| 185 | 10 | 1.15 |
| 188 | 10 | 1.15 |
| 194 | 10 | 1.00 |
| 197 | 10 | 1.00 |
| 205 | 10 | 1.00 |
| 206 | 100 | 1.00 |
| 208 | 100 | 1.08 |
| 214 | 10 | 1.15 |
| Control | - | 1.61 |

*: Each compound No. corresponds to that given in Table 5.
** Mobility: Expressed by regarding the mobility of LDL as 1.00.

Table 4

| Compound* | % of inhibition** | |
|---|---|---|
| | (Compound conc. $10^{-6}$ M) | (Compound conc. $10^{-5}$ M) |
| 118 | 99.7 | 100.0 |
| 194 | 39.1 | 99.7 |
| 205 | 100.0 | 99.9*** |
| 214 | 72.3 | 99.0 |
| Control | 0 | |

*: Each compound No. corresponds to that given in Table 5.

**:

$$\% \text{ of inhibition} = \frac{[1 - (TD_D - NSD_D)]}{(TD_C - NSD_C)} \times 100$$

$TD_C$: Total degradation when no specimen was added.

$TD_D$: Total degradation when a specimen was added.

$NSD_C$: Nonspecific degradation when no specimen was added.

$NSD_D$: Nonspecific degradation when a specimen was added.

[Each expressed in μg/mg/5 hr]

***: $10^{-5}$ M of the compound 205 was exclusively oxidized with 25 μM $CuSO_4$ while others were oxidized with 10 μM $CuSO_4$.

[Best Mode for Embodying the Invention]

## Examples

### Example 1

Synthesis of N-benzyl-3,4,5-trimethoxyaniline (compound No.1 in Table 5)

9.16 g of 3,4,5-trimethoxyaniline was dissolved in 50 ml of N,N-dimethylformamide. 6.0 ml of benzyl bromide and 7.5 ml of 1,8-diazabicyclo[5,4,0]-7-undecene (DBU) were added thereto under ice-cooling and

the resulting mixture was stirred as such overnight. The reaction mixture was poured into ice/water and extracted with ethyl acetate. The extract was washed with a saturated aqueous solution of common salt and dried over magnesium sulfate anhydride. After distilling off the solvent under reduced pressure, the residue was subjected to silica gel column chromatography and eluted with n-hexane/ethyl acetate (4 : 1). Thus 6.52 g of the target compound was obtained. m.p.: 82 ° C.

Example 2

Synthesis of N-benzylidene-3,4,5-trimethoxyaniline (intermediate)

50 g of 3,4,5-trimethoxyaniline and 31.8 g of benzaldehyde were dissolved in 200 ml of benzene. After adding a catalytic amount of p-toluenesulfonic acid, the mixture was heated under reflux for 6 hours in an azeotropic dehydrator (manufactured by Dean-Stark). After distilling off the reaction solvent under reduced pressure, the residue (solid) thus obtained was recrystallized from isopropanol. Thus 72.6 g of the target compound was obtained. m.p.: 95 ° C.

Example 3

Synthesis of N-benzyl-3,4,5-trimethoxyaniline (compound No. 1 in Table 5)

54.3 g of N-benzylidene-3,4,5-trimethoxyaniline was dissolved in 200 ml of methanol and 3.78 g of sodium borohydride was added thereto by portions under ice-cooling. The resulting mixture was stirred at room temperature for 3 hours. After distilling off the solvent under reduced pressure, water was added to the residue and stirred. The solid thus precipitated was collected by filtering under reduced pressure and dried. Thus 52.9 g of the target compound was obtained. m.p.: 83 ° C.

Example 4

Synthesis of N-benzyl-N-methyl-3,4,5-trimethoxyaniline (compound No. 2 in Table 5)

1.09 g of N-benzyl-3,4,5-trimethoxyaniline was dissolved in 40 ml of N,N-dimethylformamide. 0.37 ml of methyl iodide and 0.72 ml of 1,8-diazabicyclo[5,4,0]-7-undecene (DBU) were added thereto under ice-cooling and the resulting mixture was stirred as such overnight. The reaction mixture was poured into ice/water and extracted with ethyl acetate. The extract was washed with a saturated aqueous solution of common salt and dried over magnesium sulfate anhydride. After distilling off the solvent under reduced pressure, the residue was subjected to silica gel column chromatography and eluted with n-hexane/ethyl acetate (4 : 1). Thus 0.69 g of the target compound was obtained as an oily product. MS: 287($M^+$), 272, 91.

Example 5

Synthesis of N-benzyl-N-ethyl-3,4,5-trimethoxyaniline (compound No. 3 in Table 5)

The procedure of Example 4 was repeated except that the methyl iodide was replaced with 1.6 ml of ethyl iodide. Thus 0.48 g of the target compound was obtained as an oily product, MS: 301 ($M^+$), 286, 180, 91.

Example 6

Synthesis of N-(3,4-methylenedioxybenzylidene)-3,4,5-trimethoxyaniline (intermediate)

The procedure of Example 2 was repeated except that the benzaldehyde was replaced with 41 g of 3,4-methylene-dioxybenzaldehyde (piperonal). Thus 82.2 g of the target compound was obtained. m.p.: 112 ° C.

Example 7

Synthesis of N-(3,4-methylenedioxybenzyl)-3,4,5-trimethoxyaniline (compound No. 37 in Table 5)

The procedure of Example 3 was repeated except that the N-benzylidene-3,4,5-trimethoxyaniline was

replaced with N-(3,4-methylenedioxybenzylidene)-3,4,5-trimethoxyaniline to thereby obtain the target compound. m.p.: 78 ° C. MS: 317 ($M^+$), 181, 134

Example 8

Synthesis of N-(3,4-methylenedioxybenzyl)-N-methyl-3,4,5-trimethoxyaniline (compound No. 38 in Table 5)

0.50 g of N-(3,4-methylenedioxybenzyl)-3,4,5-trimethoxyaniline and 0.68 ml of 35% formalin were dissolved in 10 ml of acetonitrile. Then 0.20 g of sodium cyano borohydride was added thereto at room temperature and further 0.1 ml of acetic acid was added by portions. After stirring as such for 2 hours, 0.1 ml of acetic acid was added again and the resulting mixture was stirred for additional 30 minutes. To the reaction mixture, a 1 N aqueous solution of potassium hydroxide was added followed by extracting with diethyl ether. The extract was washed with a saturated aqueous solution of common salt and dried over magnesium sulfate anhydride. After distilling off the solvent under reduced pressure, the residue was subjected to silica gel column chromatography and eluted with n-hexane/ethyl acetate (3 : 1). Thus 0.47 g of target compound was obtained as an oily product. MS: 331 ($M^+$), 316, 196, 135.

Example 9

Synthesis of N-phytyl-3,4,5-trimethoxyaniline (compound No. 122 in Table 5)

1.83 g of 3,4,5-trimethoxyaniline was dissolved in 30 ml of N,N-dimethylformamide. 4.31 g of phytyl bromide and 0.58 g of sodium hydride were added thereto under ice-cooling and the resulting mixture was stirred for as such overnight. The reaction mixture was poured into ice/water and extracted with ethyl acetate. The extract was washed with a saturated aqueous solution of common salt and dried over magnesium sulfate anhydride. After distilling off the solvent under reduced pressure, the residue was subjected to silica gel column chromatography and eluted with chloroform. Thus 0.62 g of the target compound was obtained as an oily product. MS: 461 ($M^+$), 446, 183, 168.

Example 10

Synthesis of N-(1-phenylpentyl)-3,4,5-trimethoxyaniline (compound No. 77 in Table 5)

12 ml of a 2 mol/l solution of n-butyl magnesium chloride in tetrahydrofuran (THF) was dissolved in 10 ml of dry THF. Then a solution obtained by dissolving 1.64 g of N-benzylidene-3,4,5-trimethoxyaniline in 10 ml of dry THF was added dropwise thereto. After heating under reflux for 2 hours, water was slowly added thereto and the reaction mixture was extracted with ethyl acetate. The extract was washed with a saturated aqueous solution of common salt and dried over magnesium sulfate anhydride. After distilling off the solvent under reduced pressure, the residue was subjected to silica gel column chromatography and eluted with n-hexane/ethyl acetate (4 : 1). Thus 1.67 g of the target compound was obtained. m.p.: 172.3 ° C.

Example 11

Synthesis of 3',4',5'-trimethoxy-2-naphthoanilide (intermediate)

9.16 g of 3,4,5-trimethoxyaniline and 5.1 g of triethylamine were dissolved in 50 ml of chloroform. Then a solution obtained by dissolving 9.53 g of 2-naphthoyl chloride in 50 ml of chloroform was added thereto dropwise. After stirring overnight, water was added to the reaction mixture followed by extracting with chloroform. After drying over magnesium sulfate anhydride, the residue was concentrated under reduced pressure. The crude product thus obtained was recrystallized from isopropanol to thereby give 16.37 g of the target compound. m.p.: 204.9 ° C.

Example 12

Synthesis of N-naphtylmethyl-3,4,5-trimethoxyaniline (compound No. 87 in Table 5)

380 mg of lithium aluminum hydride was suspended in 30 ml of dry THF and 3',4',5'-trimethoxy-2-naphthoanilide was added thereto by portions. After heating under reflux for 3 hours, the reaction was

ceased by adding ethyl acetate and water. The insoluble matters thus precipitated were filtered through celite and then the reaction mixture was extracted with ethyl acetate and dried over magnesium sulfate anhydride. After distilling off the solvent under reduced pressure, the residue was subjected to silica gel column chromatography and eluted with n-hexane/ethyl acetate (4 : 1). thus, 2.65 g of the target compound was obtained. m.p.: 199.3° C.

Example 13

Synthesis of 2,6-di-tert-butyl-4-benzylimino-1-one (intermediate)

11 g of 2,6-di-tert-butyl-1,4-benzoquinone, 5.35 g of benzylamine and 0.5 g of p-toluenesulfonic acid were suspended in 100 ml of benzene and then heated under reflux for 5 to 6 hours with an azeotropic dehydrator )manufactured by Dean-Stark). After concentrating under reduced pressure, the reaction mixture was subjected to a silica gel column chromatography and eluted with chloroform/n-hexane. Thus the target compound was obtained. m.p.: 147 - 148° C.

Example 14

Synthesis of 2,6-di-tert-butyl-4-benzylamino-phenol (compound No. 172 in Table 5)

3 g of 2,6-di-tert-butyl-4-benzylimino-1-one was suspended in 50 ml of ethanol. After adding 1 g of sodium borohydride, the mixture was allowed to react at room temperature for 1 hour. Then it was added to a solution of benzene and water and extracted. The organic phase was washed with water twice and then a solution obtained by dissolving 1.26 g of oxalic acid in 30 ml of water was added thereto. After distilling off the solvent under reduced pressure, the residue was recrystallized from ethanol. Thus oxalate of the target compound was obtained. m.p.: 168° C (dec.).

Example 15

Synthesis of 2,6-di-tert-butyl-4-N-acetyl-N-benzylamino-phenol (compound No. 173 in Table 5)

3 ml of acetic anhydride and 3 ml of pyridine were added to the benzene phase obtained in Example 14. The resulting mixture was stirred at room temperature for 30 minutes. After concentrating the solvent under reduced pressure, the target compound was obtained. m.p.: 154° C.

Example 16

Synthesis of N-benzyl-3,5-di-tert-butyl-4-hydroxybenzylamine (compound No. 185 in Table 5)

A mixture comprising 35.1 g of 3,5-di-tert-butyl-4-hydroxybenzaldehyde, 16 g of benzylamine, 0.5 g of p-toluenesulfonic acid and 200 ml of benzene was heated under reflux for 4 hours while removing the water thus formed. Then the reaction mixture was concentrated under reduced pressure and 200 ml of methanol was added to the obtained residue. After adding 4 g of sodium borohydride under ice-cooling and stirring, the resulting mixture was stirred as such for 30 minutes and then stirred at room temperature for additional 1 hour. Then the reaction mixture was concentrated under reduced pressure and the residue was extracted with benzene and washed with water twice. The organic phase was concentrated under reduced pressure and the residue was subjected to silica gel column chromatography (eluent: chloroform). Thus 24.4 g of the target compound was obtained. The crystals thus obtained were converted into hydrochloride with the use of an ethanol/hydrochloric acid solution at room temperature. m.p.: 112 - 113° C.

Example 17

Synthesis of 4-[4'-[(trans-1,5,9-trimethyl-4,8-decadienyl)amino]phenyl]2,6-di-tert-butylphenol (compound No. 188 in Table 5)

A mixture comprising 6 g of 4-(4'-aminophenyl)-2,6-di-tert-butylphenol, 1.57 g of sodium cyano borohydride, 1.57 g of sodium sulfate anhydride, 1,2 g of acetic acid and 100 ml of dry methanol was stirred overnight at room temperature under a nitrogen gas stream. Then the reaction mixture was

concentrated under reduced pressure and the residue was extracted with benzene and washed with water twice. The organic phase was concentrated under reduced pressure and subjected to silica gel column chromatography (eluent: chloroform : n-hexane = 1 : 1). Thus 6 g of the target compound (oily) was obtained. Then the product was converted into hydrochloride by a conventional method with the use of ethanol/hydrochloric acid. m.p.: 85 - 86° C.

Example 18

Synthesis of 4-[(3,4-diacetoxyphenyl)carbonylamino]-pyridine (compound No. 199 in Table 5)

30 g of 3,4-diacetoxybenzoic acid was dissolved in 50 ml of chloroform. To the obtained solution, was added 40 g of thionyl chloride and the resulting mixture was heated under reflux for 2 hours. After the completion of the reaction, the chloroform and the excessive thionyl chloride were removed under reduced pressure and the crude product thus obtained was used in the subsequent reaction as such without purifying.

To a solution obtained by dissolving 0.95 g of 4-aminopyridine in 20 ml of chloroform, was added a solution, obtained by dissolving 2.6 g of 3,4-diacetoxybenzoic acid chloride prepared priorly in 20 ml of chloroform, dropwise under ice-cooling and stirring. After further adding 2 g of triethylamine dropwise, the resulting mixture was stirred at room temperature for 2 hours. After the completion of the reaction, the reaction mixture was washed with water twice and dried over sodium sulfate anhydride. Then the chloroform was removed under reduced pressure to thereby give 2.8 g of the target compound. m.p.: 270 - 274° C.

Example 19

Synthesis of 3-(3',4'-dimethoxyphenyl)-5-chlorobenzoisooxazol (intermediate)

To a solution obtained by dissolving 90.8 g of potassium hydroxide in 180 ml of methanol, was added a solution obtained by dissolving 15 g of 3,4-dimethoxybenzyl cyanide and 12.1 g of p-chloronitrobenzene in 120 ml of methanol. The resulting solution was stirred at room temperature for 5 hours and allowed to stand at room temperature overnight followed by adding 500 ml of water. The solid thus formed was collected by filtering, washed with water twice, dried and then purified by silica gel column chromatography (eluent: dichloromethane). Thus 4,6 g of the target compound was obtained. m.p.: 138 - 139° C..

Example 20

Synthesis of 3-(3',4'-dihydroxyphenyl)-5-chlorobenzoiso-oxazole (compound No. 205 in Table 5)

1 g of 3-(3',4'-dimethoxyphenyl)-5-chlorobenzoiso-oxazole was suspended in 20 ml of 57% hydroiodic acid and heated under reflux for 45 minutes. After the completion of the reaction, 50 ml of water was added thereto and the reaction mixture was extracted with diisopropyl ether, dried and concentrated. Thus 1.1 g of a dark brown oily product was obtained. This crude product was purified by silica gel column chromatography (eluent: chloroform) to thereby give 0.36 g of the target compound. m.p.: 187 - 190° C.

Example 21

Synthesis of 5,6-dimethyl-l-[(2E,6E)-3,7,11-trimethyldodeca-2,6,10-trienyl]-4,7-benzimidazoledione (compound No. 207 in Table 4) and synthesis of 5,6-dimethyl-1-[(2E,6Z)-3,7,11-trimethyldodeca-2,6,10-trienyl]-4,7-benzimidazoledione (compound No. 206 in Table 5)

To a mixture of 0.5 g of 5,6-dimethyl-4,7-benzimidazoledione and 50 ml of dimethylformamide, were added 1,2 g of farnesyl bromide and 0.5 ml of DBU. The mixture thus obtained was then stirred overnight. Next, it was poured into ice/water, extracted with ethyl acetate, washed with an aqueous solution of common salt and dried over sodium sulfate anhydride. After distilling off the solvent under reduced pressure, the residue was subjected to silica gel column chromatography (eluent: n-hexane/ethyl acetate). Thus 0.32 g of the target compound 207 and 0.24 g of the target compound 206 were obtained.

Rf (n-hexane : ethyl acetate = 1 : 1)
compound No. 207: 0.45, oily; and
compound No. 206: 0.54, oily.

Example 22

Synthesis of 5,6,7-trimethoxy-2,2,4-trimethyl-1,2-dihydroquinoline (compound No. 213 in Table 5)

A mixture comprising 5 g of 3,4,5-trimethoxyaniline, 2 ml of acetic acid and 80 ml of acetone was heated under reflux for 48 hours. After concentrating the reaction mixture under reduced pressure, water and ethyl acetate were added to the residue which was then extracted and dried over magnesium sulfate anhydride. After distilling off the solvent under reduced pressure, the residue was subjected to silica gel column chromatography (eluent: n-hexane : ethyl acetate = 2 : 1). Thus 6.52 g of the target compound was obtained. m.p.: 116 - 119 °C.

The compounds shown in Table 5 (compounds No. 1 to No. 215) were synthesized by methods similar to those described in Examples 1 to 22.

Table 5

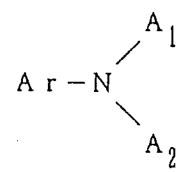

| compound | Ar | $A_1$ | $A_2$ | $^1$H-NMR ($CDCl_3$, δ value)‡ | m.p. (℃)‡‡ |
|---|---|---|---|---|---|
| 1 | 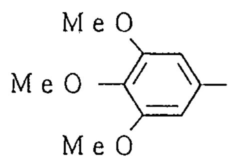 | 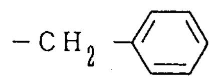 | H | 3.70(9H, s), 3.80(1H, b), 4.22(2H, s), 5.78(2H, s), 7.21(5H, s) | 83 |
| 2 | 〃 | 〃 | Me | 2.93(3H, s), 3.72(9H, s), 4.40(2H, s), 5.90(2H, s), 7.17(5H, s) | (147) |
| 3 | 〃 | 〃 | Et | 1.19(3H, t, J=7.0Hz), 3.40(2H, q, J=7.0Hz), 3.71(9H, s), 4.42(2H, s), 5.87(2H, s), 7.20(5H, s) | oily |
| 4 | 〃 | 〃 | 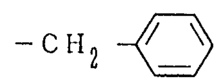 | 3.62(6H, s), 3.71(3H, s), 4.53(4H, s), 5.90(2H, s), 7.20(10H, s) | (167) |
| 5 | 〃 | 〃 | $(CH_2)_3OH$ | 1.60-2.10(4H, m), 3.32-3.85(3H, m), 3.67(9H, s), 4.38(2H, s), 5.88(2H, s), 7.10(5H, s) | <273dec> |

Table 5 (contd.)

| compound | Ar | $A_1$ | $A_2$ | $^1$H-NMR ($CDCl_3$, δvalue‡) | m.p.(℃)‡‡ |
|---|---|---|---|---|---|
| 6 | 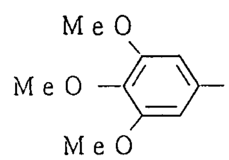 | 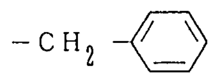 | $-(CH_2)_2COOMe$ | 2.60(2H, t, J=7.0Hz), 3.60(3H, s), 3.69(2H, t, J=7.0Hz), 3.70(9H, s), 4.45(2H, s), 5.91(2H, s), 7.18(5H, s) | oily |
| 7 | 〃 | 〃 | $-(CH_2)_2COONa$ | 2.53(2H, dd, J=8.0Hz, 5.0Hz), 3.50-3.80(2H, dd, J=8.0Hz, 5.0Hz), 3.67 (9H, s), 4.43(2H, s), 5.88(2H, s), 7.13(5H, s) | 126 |
| 8 | 〃 | 〃 | 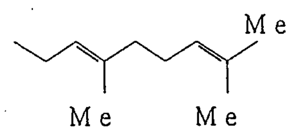 | 1.55-1.78(9H, m), 1.95-2.20 (4H, m), 3.71(9H, s), 3.90(2H, d, J=6.0Hz), 4.40(2H, s), 4.85-5.40 (2H, m), 5.87(2H, s), 7.18(5H, s) | oily |
| 9 | 〃 | 〃 | 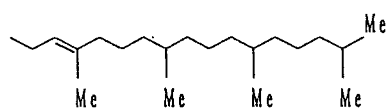 | 0.87(12H, d, J=6.0Hz), 1.05-2.20 (21H, m), 1.67(3H, s), 3.75(9H, s), 3.97(2H, d, J=6.0Hz), 4.50(2H, s), 5.32(1H, t, J=6.0Hz), 5.98(2H, s), 7.30(5H, s) | oily |

Table 5 (contd.)

| compound | Ar | $A_1$ | $A_2$ | $^1$H-NMR ($CDCl_3$, δvalue) | m.p. (°C) |
|---|---|---|---|---|---|
| 10 | 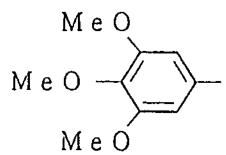 | 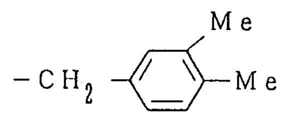 | H | 2.22 (6H, s), 3.71 (3H, s), 3.74 (6H, s), 3.80 (1H, b), 4.16 (2H, s), 5.82 (2H, s), 7.04 (3H, s) | (171) |
| 11 | 〃 | 〃 | Me | 2.21 (6H, s), 2.91 (3H, s), 3.75 (9H, s), 4.34 (2H, s), 5.92 (2H, s), 6.95 (3H, s) | (157) |
| 12 | 〃 | 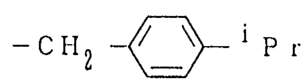 | H | 1.22 (6H, d, J=7.0Hz), 2.88 (1H, m), 3.73 (9H, s), 3.80 (1H, b), 4.20 (2H, s), 5.81 (2H, s), 7.18 (4H, s) | (183) |
| 13 | 〃 | 〃 | Me | 1.20 (6H, d, J=7.0Hz), 2.85 (1H, m), 2.90 (3H, s), 3.69 (3H, s), 3.71 (6H, s), 4.35 (2H, s), 5.87 (2H, s), 7.04 (4H, s) | (102) |
| 14 | 〃 | 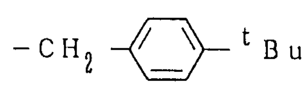 | H | 1.29 (9H, s), 3.69 (3H, s), 3.70 (6H, s), 3.78 (1H, b), 4.16 (2H, s), 5.76 (2H, s), 7.18 (4H, s) | (176) |
| 15 | 〃 | 〃 | Me | 1.29 (9H, s), 2.92 (3H, s), 3.67 (3H, s), 3.70 (6H, s), 4.34 (2H, s), 5.86 (2H, s), 7.02 (2H, d, J=9.0Hz), 7.24 (2H, d, J=9.0Hz) | (152) |

Table 5 (contd.)

| compound | Ar | $A_1$ | $A_2$ | $^1H$-NMR ($CDC\ell_3$, δvalue‡) | m.p. (°C) ‡‡ |
|---|---|---|---|---|---|
| 16 | 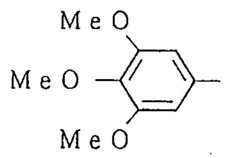 | 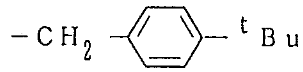 | 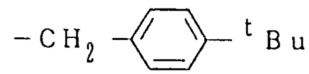 | 1.28 (18H, s), 3.62 (6H, s), 3.70 (3H, s) 4.48 (4H, s), 5.89 (2H, s), 7.06 (4H, d, J=9.0Hz), 7.26 (4H, d, J=9.0Hz) | (171) |
| 17 | 〃 | 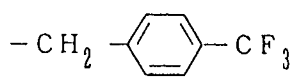 | H | 3.71 (9H, s), 3.90 (1H, b), 4.33 (2H, s), 5.79 (2H, s), 7.47 (4H, s) | (234dec) |
| 18 | 〃 | 〃 | Me | 2.95 (3H, s), 3.72 (9H, s), 4.43 (2H, s), 5.85 (2H, s), 7.22 (2H, d, J=9.0Hz), 7.48 (2H, d, J=9.0Hz) | (92.5) |
| 19 | 〃 | 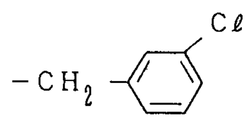 | H | 3.70 (3H, s), 3.72 (6H, s), 3.90 (1H, b), 4.22 (2H, s), 5.79 (2H, s), 7.10-7.35 (4H, m) | (174) |
| 20 | 〃 | 〃 | Me | 2.95 (3H, s), 3.72 (3H, s), 3.75 (6H, s), 4.38 (2H, s), 5.88 (2H, s), 7.05-7.22 (4H, m) | (167) |

Table 5 (contd.)

| compound | Ar | $A_1$ | $A_2$ | $^1H-NMR$ ($CDCl_3$, δvalue‡) | m.p. (℃) ‡‡ |
|---|---|---|---|---|---|
| 21 | 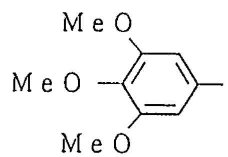 | 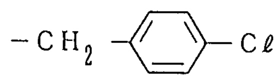 | H | 3.72(9H, s), 3.90(1H, b), 4.22(2H, s), 5.78(2H, s), 7.21(4H, s) | (224dec) |
| 22 | 〃 | 〃 | Me | 2.90(3H, s), 3.69(3H, s), 3.71(6H, s), 4.34(2H, s), 5.85(2H, s), 7.11(4H, s) | (167) |
| 23 | 〃 | 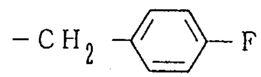 | H | 3.71(9H, s), 3.84(1H, b), 4.20(2H, s), 5.77(2H, s), 6.76-7.36(4H, m) | (164) |
| 24 | 〃 | 〃 | Me | 2.93(3H, s), 3.73(3H, s), 3.76(6H, s), 4.40(2H, s), 5.91(2H, s), 6.70-7.35 (4H, m) | (163) |
| 25 | 〃 | 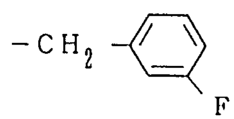 | H | 3.77(9H, s), 4.30(2H, s), 5.85(2H, s), 6.78-7.37(4H, m) | (174.9) |
| 26 | 〃 | 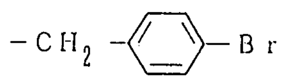 | H | 3.75(9H, s), 4.23(2H, s), 5.83(2H, s), 7.18(2H, d, J=9.0Hz), 7.41(2H, d, J=9.0Hz) | (153) |

Table 5 (contd.)

| compound | Ar | $A_1$ | $A_2$ | ¹H−NMR ($CDCl_3$, δ value)‡ | m.p. ‡‡ (°C) |
|---|---|---|---|---|---|
| 27 | 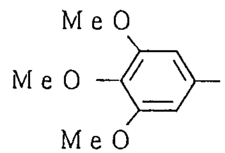 | $-CH_2-$ 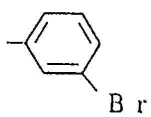 | H | 3.73 (9H, s), 4.23 (2H, s), 5.83 (2H, s), 7.08−7.48 (4H, m) | (188.8) |
| 28 | 〃 | $-CH_2-$ 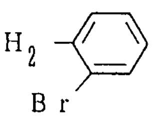 | H | 3.73 (3H, s), 3.77 (6H, s), 4.35 (2H, s), 5.83 (2H, s), 7.07−7.63 (4H, m) | (176.9) |
| 29 | 〃 | $-CH_2-$ 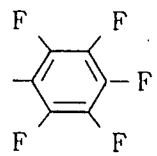 | H | 3.68 (3H, s), 3.76 (6H, s), 4.39 (2H, s), 5.85 (2H, s) | (>200dec) |
| 30 | 〃 | 〃 | $-(CH_2)_3-$ 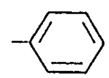 | 1.80−2.17 (2H, m), 2.67 (2H, t, J=7.0Hz), 3.33 (2H, t, J=8.0Hz), 3.77 (9H, s), 4.48 (2H, s), 6.00 (2H, s), 7.22 (5H, s) | oily |

Table 5 (contd.)

| compound | Ar | $A_1$ | $A_2$ | ¹H−NMR (CDCℓ₃, δ value)‡ | m.p. (°C) ‡‡ |
|---|---|---|---|---|---|
| 31 | 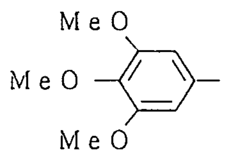 | 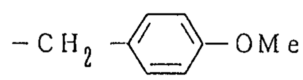 | H | 3. 75 (12H, s), 3. 83 (1H, b), 4. 17 (2H, s), 5. 80 (2H, s), 6. 78 (2H, d, J=8. 5Hz), 7. 22 (2H, d, J=8. 5Hz) | (192dec) |
| 32 | 〃 | 〃 | Me | 2. 92 (3H, s), 3. 73 (3H, s), 3. 75 (9H, s), 4. 35 (2H, s), 5. 91 (2H, s), 6. 76 (2H, d, J=8. 5Hz), 7. 10 (2H, d, J=8. 5Hz) | (153) |
| 33 | 〃 | 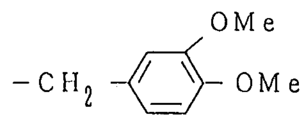 | H | 3. 73 (3H, s), 3. 76 (6H, s), 3. 84 (6H, s), 3. 90 (1H, b), 4. 18 (2H, s), 5. 82 (2H, s), 6. 77−6. 92 (3H, m) | 131 |
| 34 | 〃 | 〃 | Me | 2. 92 (3H, s), 3. 72 (3H, s), 3. 74 (6H, s), 3. 76 (3H, s), 3. 79 (3H, s), 4. 34 (2H, s), 5. 93 (2H, s), 6. 70 (3H, s) | 90 |
| 35 | 〃 | 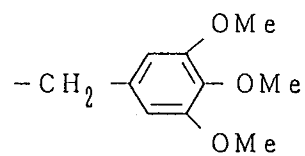 | H | 3. 71 (3H, s), 3. 75 (6H, s), 3. 80 (9H, s), 3. 88 (1H, b), 4. 17 (2H, s), 5. 83 (2H, s), 6. 54 (2H, s) | (184) |

Table 5 (contd.)

| compound | Ar | $A_1$ | $A_2$ | $^1$H-NMR ($CDCl_3$, δ value)‡ | m.p. (℃) ‡‡ |
|---|---|---|---|---|---|
| 36 | 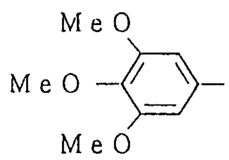 | 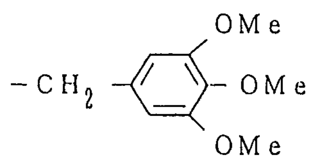 | Me | 2.93 (3H, s), 3.76 (18H, s), 4.35 (2H, s), 5.95 (2H, s), 6.41 (2H, s) | (171) |
| 37 | 〃 | 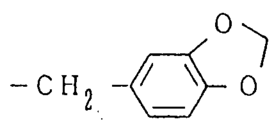 | H | 3.68 (3H, s), 3.70 (6H, s), 3.87 (1H, b), 4.12 (2H, s), 5.77 (2H, s), 5.83 (2H, s), 6.67-6.80 (3H, m) | 78 |
| 38 | 〃 | 〃 | Me | 2.91 (3H, s), 3.70 (3H, s), 3.73 (6H, s), 4.30 (2H, s), 5.82 (2H, s), 5.88 (2H, s), 6.63 (3H, s) | (162) |
| 39 | 〃 | 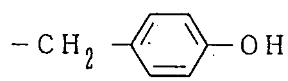 | H | 3.23 (1H, b), 3.57 (3H, s), 3.67 (6H, s), 4.06 (2H, d, J=5.0Hz), 5.23 (1H, t, J=5.0Hz), 5.80 (2H, s), 6.60 (2H, d, J=9.0Hz), 7.04 (2H, d, J=9.0Hz) ② | 149 |
| 40 | 〃 | 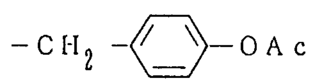 | H | 2.29 (3H, s), 3.70 (1H, b), 3.76 (9H, s), 4.26 (2H, s), 5.82 (2H, s), 7.00 (2H, d, J=9.0Hz), 7.34 (2H, d, J=9.0Hz) | (200dec) |

Table 5 (contd.)

| compound | Ar | $A_1$ | $A_2$ | $^1$H-NMR ($CDCl_3$, δ value)‡ | m.p.‡‡ (°C) |
|---|---|---|---|---|---|
| 41 | 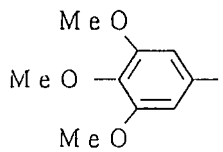 | 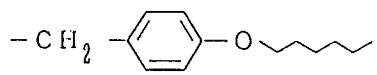 | H | 0.88 (3H, t, J=6.0Hz), 1.10-1.95 (8H, m), 3.03 (1H, b), 3.68 (9H, s), 3.85 (2H, t, J=6.0Hz), 4.10 (2H, s), 5.73 (2H, s), 6.68 (2H, d, J=9.0Hz), 7.10 (2H, d, J=9.0Hz) | (171.1) |
| 42 | 〃 | 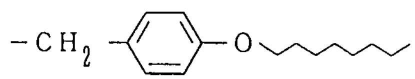 | H | 0.87 (3H, t, J=6.0Hz), 1.05-1.95 (12H, m), 3.70 (9H, s), 3.88 (2H, t, J=7.0Hz), 4.13 (2H, s), 5.78 (2H, s), 6.77 (2H, d, J=9.0Hz), 7.15 (2H, d, J=9.0Hz) | (165.3) |
| 43 | 〃 | 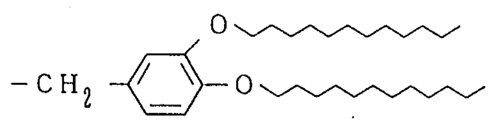 | H | 0.88 (6H, t, J=6.0Hz), 1.10-2.10 (40H, m), 3.82 (3H, s), 3.86 (6H, s), 4.04 (4H, m), 4.06 (2H, s), 6.40 (2H, s), 6.80-7.57 (3H, m) | (189.3) |
| 44 | 〃 | 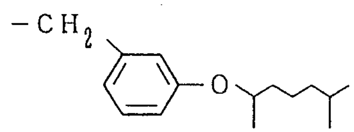 | H | 0.85 (6H, d, J=6.0Hz), 1.10-1.75 (7H, m), 1.25 (3H, d, J=6.0Hz), 3.73 (9H, s), 4.20 (2H, s), 4.27 (1H, m), 5.83 (2H, s), 6.60-7.35 (4H, m) | (106.7) |

Table 5 (contd.)

| compound | Ar | $A_1$ | $A_2$ | $^1H$-NMR ($CDCl_3$, δ value)‡ | m.p. (℃) ‡‡ |
|---|---|---|---|---|---|
| 45 | 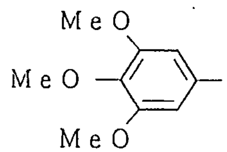 | 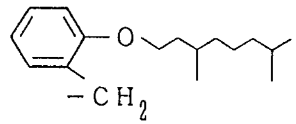 | H | 0.85 (9H, d, J=7.0Hz), 1.10-1.90 (10H, m), 3.75 (9H, s), 4.03 (2H, t, J=7.0Hz), 4.28 (2H, s), 5.86 (2H, s), 6.65-7.40 (4H, m) | (120.3) |
| 46 | 〃 | 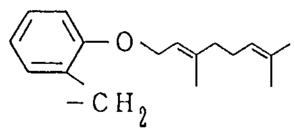 | H | 1.60 (3H, s), 1.67 (3H, s), 1.72 (3H, s), 2.00-2.20 (4H, m), 3.73 (3H, s), 3.78 (6H, s), 4.30 (2H, s), 4.58 (2H, d, J=6.0Hz), 5.09 (1H, m), 5.49 (1H, t, J=6.0Hz), 5.88 (2H, s), 6.70-7.40 (4H, m) | (123.8) |
| 47 | 〃 | 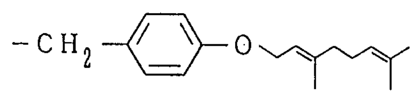 | H | 1.55-1.77 (9H, m), 2.00-2.18 (4H, m), 3.70 (3H, s), 3.72 (6H, s), 4.15 (2H, s), 4.48 (2H, d, J=7.0Hz), 5.04 (1H, b), 5.43 (1H, t, J=7.0Hz), 5.80 (2H, s), 6.79 (2H, d, J=9.0Hz), 7.20 (2H, d, J=9.0Hz) | (147.7) |
| 48 | 〃 | 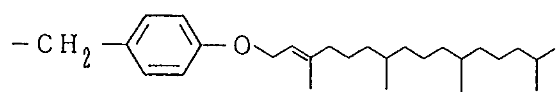 | H | 0.85 (12H, d, J=6.0Hz), 1.00-2.20 (21H, m), 1.72 (3H, s), 3.75 (9H, s), 4.18 (2H, s), 4.50 (2H, d, J=7.0Hz), 5.45 (1H, t, J=7.0Hz), 5.84 (2H, s), 6.84 (2H, d, J=9.0Hz), 7.25 (2H, d, J=9.0Hz) | (215.1) |

Table 5 (contd.)

| compound | Ar | $A_1$ | $A_2$ | $^1H$-NMR ($CDCl_3$, $\delta$ value)‡ | m.p. (℃)‡‡ |
|---|---|---|---|---|---|
| 49 | 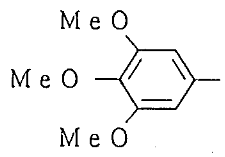 | 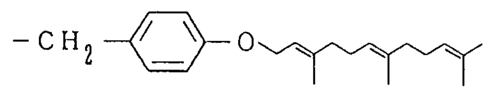 | H | 1.58-1.78 (12H, m), 1.98-2.22 (8H, m), 3.53 (1H, b), 3.75 (9H, s), 4.18 (2H, s), 4.50 (2H, d), 4.92-5.22 (3H, m), 5.83 (2H, s), 6.83 (2H, d, J=9.0Hz), 7.23 (2H, d, J=9.0Hz) | (205.6) |
| 50 | 〃 | 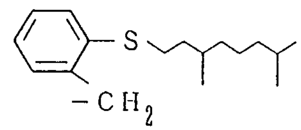 | H | 0.87 (9H, d, J=6.0Hz), 1.05-1.95 (10H, m), 2.93 (2H, t, J=7.0Hz), 3.72 (3H, s), 3.75 (6H, s), 4.00 (1H, b), 4.36 (2H, s), 5.83 (2H, s), 7.00-7.43 (4H, m) | (88) |
| 51 | 〃 | 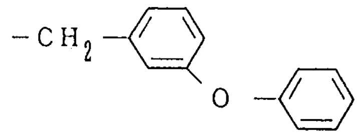 | H | 3.73 (9H, s), 4.22 (2H, s), 5.77 (2H, s), 6.78-7.23 (9H, m) | (146.8) |
| 52 | 〃 | 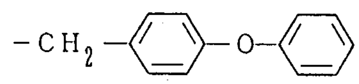 | H | 3.40 (1H, b), 3.70 (3H, s), 3.73 (6H, s), 4.18 (2H, s), 5.80 (2H, s), 6.89 (2H, d, J=9.0Hz), 7.15 (5H, s), 7.26 (2H, d, J=9.0Hz) | (196.5) |
| 53 | 〃 | 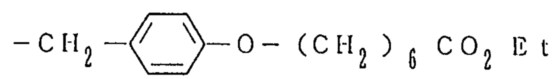 | H | 1.23 (3H, t, J=7.0Hz), 1.35-1.72 (10H, m), 2.30 (2H, t), 3.74 (3H, s), 3.77 (3H, s), 4.00 (2H, q, J=7.0Hz), 4.18 (2H, s), 5.85 (2H, s), 6.82 (2H, d, J=8.0Hz), 7.24 (2H, d, J=8.0Hz) | (151.7) |

Table 5 (contd.)

| compound | Ar | $A_1$ | $A_2$ | $^1$H-NMR ($CDCl_3$, δ value) | m.p. (℃) |
|---|---|---|---|---|---|
| 54 | 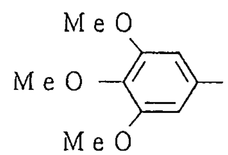 | 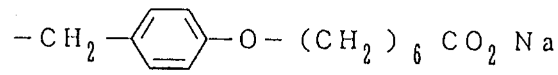 | H | 1.33-1.82 (10H, m), 2.05 (2H, t), 3.73 (3H, s), 3.78 (6H, s), 4.20 (2H, s), 5.90 (2H, s), 6.85 (2H, d, J=8.0Hz), 7.27 (2H, d, J=8.0Hz) | 258.1 |
| 55 | 〃 | 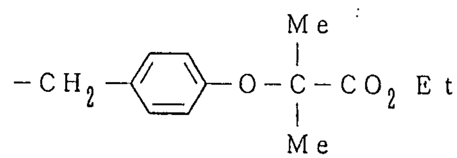 | H | 1.23 (3H, t, J=7.0Hz), 1.56 (6H, s), 3.72 (9H, s), 4.16 (2H, s), 4.18 (2H, q, J=7.0Hz), 5.80 (2H, s), 6.75 (2H, d, J=9.0Hz), 7.18 (2H, d, J=9.0Hz) | (154.4) |
| 56 | 〃 | 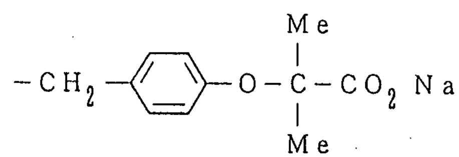 | H | 1.26 (6H, s), 3.71 (9H, s), 4.12 (2H, s), 5.80 (2H, s), 6.75 (2H, d, J=9.0Hz), 7.10 (2H, d, J=9.0Hz) | 103.1 |
| 57 | 〃 | 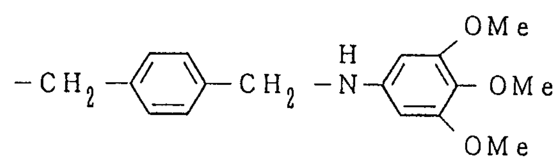 | H | 3.72 (18H, s), 4.23 (4H, s), 5.78 (4H, s), 7.17-7.28 (4H, m) | (221.6) |

Table 5 (contd.)

| compound | Ar | $A_1$ | $A_2$ | $^1H$-NMR ($CDCl_3$, δ value) | m.p. (℃) ‡‡ |
|---|---|---|---|---|---|
| 58 | 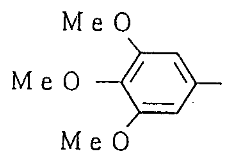 | 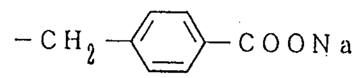 | H | 3.60 (3H, s), 3.65 (6H, s), 4.23 (2H, b), 5.83 (2H, s), 7.26 (2H, d, J=7.5Hz), 7.79 (2H, d, J=7.5Hz) ① | >300 |
| 59 | 〃 | 〃 | Me | 3.00 (3H, s), 3.70 (3H, s), 3.74 (6H, s), 4.48 (2H, b), 5.92 (2H, s), 7.16 (2H, d, J=8.0Hz), 7.85 (2H, d, J=8.0Hz) ① | >300 |
| 60 | 〃 | 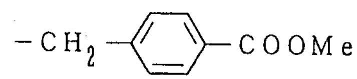 | H | 3.68 (9H, s), 3.83 (3H, s), 4.00 (1H, b), 4.29 (2H, s), 5.74 (2H, s), 7.29 (2H, d, J=8.0Hz), 7.87 (2H, d, J=8.0Hz) | (197dec) |
| 61 | 〃 | 〃 | Me | 2.99 (3H, s), 3.73 (9H, s), 3.86 (3H, s), 4.48 (2H, s), 5.88 (2H, s), 7.25 (2H, d, J=8.0Hz), 7.92 (2H, d, J=8.0Hz) | 85-86 |
| 62 | 〃 | 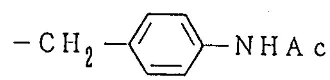 | H | 2.08 (3H, s), 3.65 (3H, s), 3.72 (6H, s), 4.17 (2H, d, J=5.5Hz), 4.71 (1H, b), 5.81 (2H, s), 7.18 (2H, d, J=9.0Hz), 7.48 (2H, d, J=9.0Hz), 9.30 (1H, b) ② | 169-170 |

Table 5 (contd.)

| compound | Ar | $A_1$ | $A_2$ | $^1H$-NMR ($CDCl_3$, δ value) | m.p. (°C) ‡‡ |
|---|---|---|---|---|---|
| 63 | 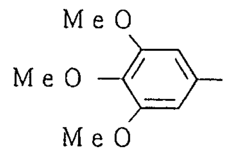 | 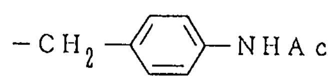 | Me | 2.01 (3H, s), 2.90 (3H, s), 3.53 (3H, s), 3.65 (6H, s), 4.35 (2H, s), 5.87 (2H, s), 7.03 (2H, d, J=9.0Hz), 7.37 (2H, d, J=9.0Hz) ③ | 141 |
| 64 | 〃 | 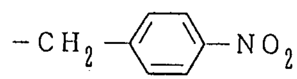 | H | 3.68 (9H, s), 4.16 (1H, b), 4.38 (2H, s), 5.76 (2H, s), 7.43 (2H, d, J=9.0Hz), 8.09 (2H, d, J=9.0Hz) | (174) |
| 65 | 〃 | 〃 | Me | 3.00 (3H, s), 3.74 (3H, s), 3.76 (6H, s), 4.53 (2H, s), 5.89 (2H, s), 7.38 (2H, d, J=8.5Hz), 8.13 (2H, d, J=8.5Hz) | (173) |
| 66 | 〃 | 〃 | 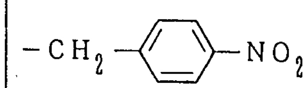 | 3.62 (6H, s), 3.70 (3H, s), 4.60 (4H, s), 5.80 (2H, s), 7.31 (4H, d, J=8.5Hz), 8.07 (4H, d, J=8.5Hz) | 178 |

Table 5 (contd.)

| compound | Ar | $A_1$ | $A_2$ | ¹H-NMR ($CDCl_3$, δ value)‡ | m.p. (℃)‡‡ |
|---|---|---|---|---|---|
| 67 | 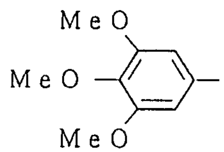 | 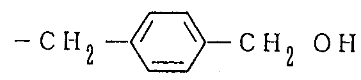 | H | 3.71(9H, s), 4.21(2H, s), 4.59(2H, s), 5.78(2H, s), 7.23(4H, s) | (194) |
| 68 | 〃 | 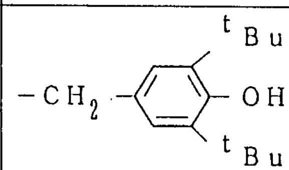 | H | 1.41(18H, s), 3.70(3H, s), 3.75(6H, s), 4.10(2H, s), 5.10(1H, s), 5.84(2H, s), 7.14(2H, s) | <183> |
| 69 | 〃 | 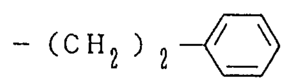 | H | 2.82(2H, t, J=6.0Hz), 3.28(2H, t, J=6.0Hz), 3.69(9H, s), 5.74(2H, s), 7.12(5H, s) | (172.6) |
| 70 | 〃 | 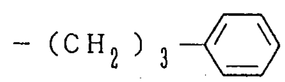 | H | 1.90(2H, m, J=7.0Hz), 2.70(2H, t, J=7.0Hz), 3.07(2H, t, J=7.0Hz), 3.72(9H, s), 5.71(2H, s), 7.15(5H, s) | (207.2) |
| 71 | 〃 | 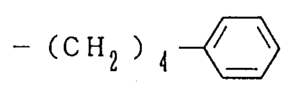 | H | 1.55-1.82(4H, m), 2.63(2H, t, J=6.0Hz), 3.03(2H, t, J=6.0Hz), 3.73(3H, s), 3.77(6H, s), 5.77(2H, s), 7.15(5H, s) | (159.0) |

Table 5 (contd.)

| compound | Ar | $A_1$ | $A_2$ | $^1$H−NMR ($CDCl_3$, δ value)* | m.p. (°C) ** |
|---|---|---|---|---|---|
| 72 | 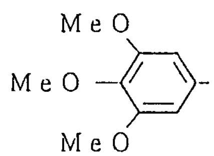 | $-(CH_2)_6-$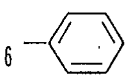 | H | 1. 33-1. 75 (8H, m), 2. 58 (2H, t, J=6. 0Hz), 3. 03 (2H, t, J=6. 0Hz), 3. 73 (3H, s), 3. 78 (3H, s), 5. 78 (2H, s), 7. 16 (5H, s) | (163. 3) |
| 73 | 〃 | 〃 | $-(CH_2)_6-$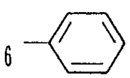 | 1. 20-1. 75 (16H, m), 2. 58 (4H, t, J=6. 0Hz), 3. 18 (4H, t, J=6. 0Hz), 3. 77 (9H, s), 5. 85 (2H, s), 7. 15 (10H, s) | (101. 9) |
| 74 | 〃 | $-(CH_2)_8-$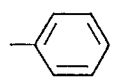 | H | 1. 27-1. 83 (12H, m), 2. 60 (2H, t, J=6. 0Hz), 3. 05 (2H, t, J=6. 0Hz), 3. 75 (3H, s), 3. 83 (6H, s), 5. 83 (2H, s), 7. 20 (5H, s) | (124. 4) |
| 75 | 〃 | $-(CH_2)_3-$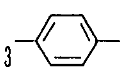$-CH_2OH$ | H | 1. 37 (1H, b), 1. 80 (2H, b), 2. 19 (2H, m), 2. 60 (2H, t, J=7. 5Hz), 3. 23 (2H, t, J=7. 5Hz), 3. 79 (9H, s), 4. 59 (2H, s), 6. 75 (2H, s), 7. 01 (2H, d, J=8. 5Hz), 7. 18 (2H, d, J=8. 5Hz) ⑤ | (165) |
| 76 | 〃 | 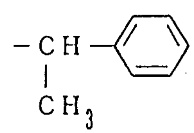 | H | 1. 47 (3H, d, J=7. 0Hz), 3. 61 (6H, s), 3. 65 (3H, s), 3. 90 (1H, b), 4. 36 (1H, q, J=7. 0Hz), 5. 68 (2H, s), 7. 24 (5H, s) | (173) |

Table 5 (contd.)

| compound | Ar | $A_1$ | $A_2$ | $^1$H-NMR ($CDCl_3$, δ value)‡ | m.p. (°C) ‡‡ |
|---|---|---|---|---|---|
| 77 | 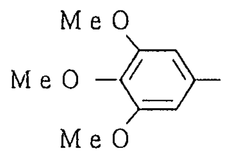 | 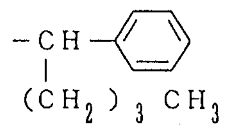 | H | 0.85 (3H, t), 1.25-1.95 (6H, m), 3.60 (6H, s), 3.67 (3H, s), 4.20 (1H, t), 5.73 (3H, s), 7.22 (5H, s) | (172.3) |
| 78 | 〃 | 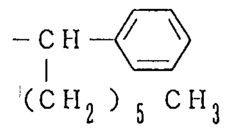 | H | 0.88 (3H, t), 1.08-1.83 (10H, m), 3.70 (9H, s), 4.07-4.33 (1H, m), 5.75 (2H, s), 7.32 (5H, s) | (194.2) |
| 79 | 〃 | 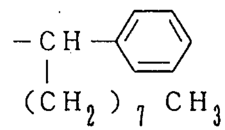 | H | 0.87 (3H, t), 1.08-1.95 (14H, m), 3.68 (9H, s), 4.03-4.33 (1H, m), 5.72 (2H, s), 7.28 (5H, s) | (185.3) |
| 80 | 〃 | 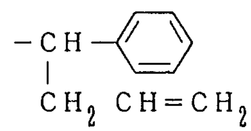 | H | 3.64 (6H, s), 3.68 (3H, s), 4.2-4.41 (3H, m), 5.23 (2H, d), 5.23-6.00 (1H, m), 5.70 (2H, s), 7.27 (5H, s) | (200.0) |
| 81 | 〃 | 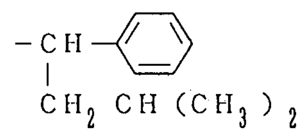 | H | 0.83 (6H, d), 1.42-1.73 (3H, m), 3.55 (6H, s), 3.58 (3H, s), 4.03-4.30 (1H, m), 5.57 (2H, s), 7.10 (5H, s) | (205.9) |

Table 5 (contd.)

| compound | Ar | $A_1$ | $A_2$ | $^1H-NMR$ ($CDCl_3$, δ value*) | m.p.** (℃) |
|---|---|---|---|---|---|
| 82 | 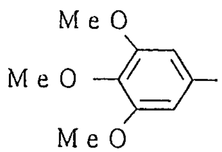 | 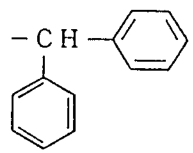 | H | 3.60 (6H, s), 3.70 (3H, s), 4.22 (1H, s), 5.75 (2H, s), 7.23 (10H, s) | (288.0) |
| 83 | 〃 | 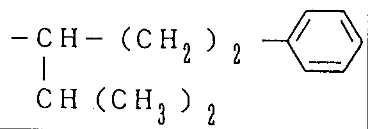 | H | 0.92 (6H, d), 1.63-2.03 (3H, m), 2.70 (2H, t, J=6.0Hz), 3.02-3.40 (1H, m), 3.75 (9H, s), 5.72 (2H, s), 7.13 (5H, s) | (192.6) |
| 84 | 〃 | 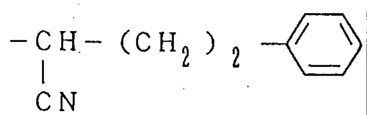 | H | 2.19 (2H, q, J=7.0Hz), 2.88 (2H, t, J=7.0Hz), 3.71 (9H, s), 3.85 (1H, m), 5.79 (2H, s), 7.16 (5H, s) | (190.6) |
| 85 | 〃 | 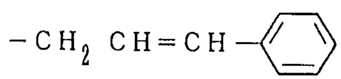 | H | 3.69 (3H, s), 3.73 (6H, s), 3.86 (2H, s), 5.82 (2H, s), 6.20-6.70 (2H, m), 7.20 (5H, m) | (147) |
| 86 | 〃 | 〃 | 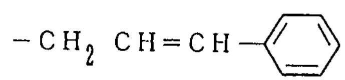 | 3.76 (3H, s), 3.80 (6H, s), 4.16 (4H, d, J=5.0Hz), 6.36-6.77 (4H, m), 7.02 (4H, s), 7.18 (10H, s) | (133) |

Table 5 (contd.)

| compound | Ar | $A_1$ | $A_2$ | $^1$H-NMR ($CDCl_3$, δ value)‡ | m.p. (℃)‡‡ |
|---|---|---|---|---|---|
| 87 | 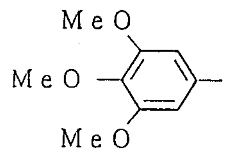 | 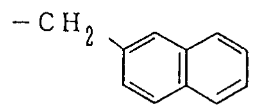 | H | 3. 73 (9H, s), 4. 41 (2H, s), 5. 88 (2H, s), 7. 28-7. 90 (7H, m) | (199. 3) |
| 88 | 〃 | 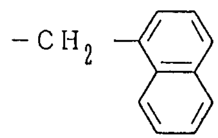 | H | 3. 72 (9H, s), 3. 80 (1H, b), 4. 60 (2H, s), 5. 82 (2H, s), 7. 28-8. 10 (7H, m) | (198dec) |
| 89 | 〃 | 〃 | Me | 2. 95 (3H, s), 3. 65 (6H, s), 3. 70 (3H, s), 4. 80 (2H, s), 5. 87 (2H, s), 7. 18-7. 98 (7H, m) | 126 |
| 90 | 〃 | 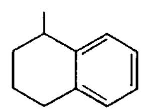 | H | 1. 64-2. 12 (4H, m), 2. 77 (2H, m), 3. 70 (3H, s), 3. 74 (6H, s), 4. 49 (1H, m), 5. 80 (2H, s), 6. 93-7. 40 (5H, m) | (138-140) |
| 91 | 〃 | 〃 | Me | 1. 65-2. 21 (4H, m), 2. 63 (3H, s), 2. 78 (2H, m), 3. 75 (3H, s), 3. 78 (6H, s), 4. 95 (1H, m), 6. 00 (2H, s), 7. 00-7. 30 (4H, m) | 99 |

Table 5 (contd.)

| compound | Ar | $A_1$ | $A_2$ | $^1$H−NMR ($CDCl_3$, δ value)‡ | m.p. (℃)‡‡ |
|---|---|---|---|---|---|
| 92 | 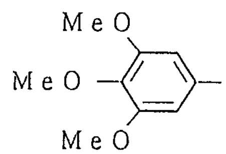 | 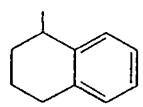 | Et. | 1.16(3H, t, J=7.0Hz), 1.82−2.20 (4H, m), 2.77(2H, m), 3.13(2H, q, J=7.0Hz), 3.70(9H, s), 4.82(1H, m), 5.90(2H, s), 6.93−7.30(4H, m) | (202) |
| 93 | 〃 | 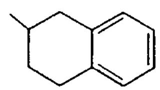 | H | 2.10−3.67(7H, m), 3.78(9H, s), 5.87(2H, s), 7.07(4H, s) | (219.3) |
| 94 | 〃 | $-CH_2$ 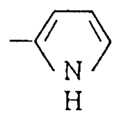 | H | 3.73(3H, s), 3.77(6H, s), 4.27(2H, s), 5.88(2H, s), 6.07−6.12(2H, m), 6.61−6.78(1H, m) | 120.9 |
| 95 | 〃 | $-CH_2$ 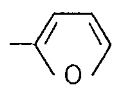 | H | 3.72(3H, s), 3.75(6H, s), 4.23(2H, s), 5.85(2H, s), 6.12−6.28(2H, m), 7.27(1H, b) | (>200dec) |
| 96 | 〃 | 〃 | $-CH_2CH=CH_2$ | 3.75(3H, s), 3.82(6H, s), 3.98−4.16(2H, m), 4.40(2H, s), 5.10−5.33(2H, m), 5.60−5.93(1H, m), 6.05(2H, s), 6.13−6.42(2H, m), 7.35(1H, b) | (144.4) |

Table 5 (contd.)

| compound | Ar | $A_1$ | $A_2$ | $^1H$-NMR ($CDCl_3$, $\delta$ value)[‡] | m.p. (℃)[‡‡] |
|---|---|---|---|---|---|
| 97 | 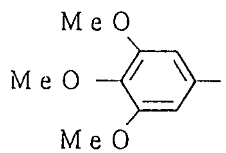 | 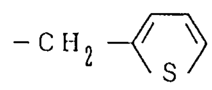 | H | 3.75 (3H, s), 3.78 (6H, s), 4.45 (2H, s), 5.88 (2H, s), 6.95 (1H, b), 7.13-7.25 (2H, m) | (221.3) |
| 98 | 〃 | 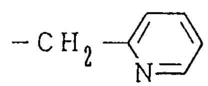 | H | 3.75 (9H, s), 4.42 (2H, s), 5.90 (2H, s), 7.02-7.75 (3H, m), 8.45-8.62 (1H, m) | (188.6) |
| 99 | 〃 | 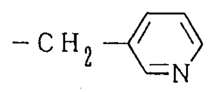 | H | 3.78 (9H, s), 4.32 (2H, s), 5.88 (2H, s), 7.13-7.35 (1H, m), 7.58-7.78 (1H, m), 8.42-8.63 (2H, m) | (206.1) |
| 100 | 〃 | 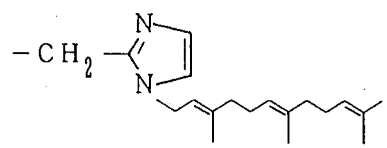 | H | 1.55-1.80 (12H, m), 1.98-2.18 (8H, m), 3.75 (3H, s), 3.80 (6H, s), 4.32 (2H, s), 4.53 (2H, d), 4.97-5.37 (3H, m), 5.95 (2H, s), 6.88 (2H, d) | (117.3) |
| 101 | 〃 | 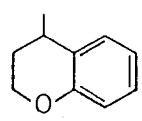 | H | 2.07 (2H, m), 3.70 (3H, s), 3.74 (6H, s), 4.18 (2H, t, J=6.0Hz), 4.50 (1H, b), 5.82 (2H, s), 6.63-7.35 (4H, m) | (227.4) |

Table 5 (contd.)

| compound | Ar | $A_1$ | $A_2$ | $^1$H-NMR ($CDCl_3$, δ value)* | m.p. (℃)** |
|---|---|---|---|---|---|
| 102 | 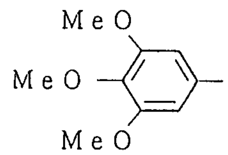 | 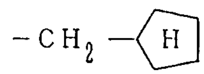 | H | 1.05-2.10 (9H, m), 2.97 (2H, d, J=7.0Hz), 3.70 (3H, s), 3.77 (6H, s), 5.78 (2H, s) | (205.2) |
| 103 | 〃 | 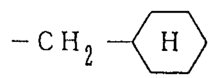 | H | 0.70-2.05 (11H, m), 2.93 (2H, d, J=6.0Hz), 3.60 (1H, b), 3.76 (3H, s), 3.82 (6H, s), 5.84 (2H, s) | (202.7) |
| 104 | 〃 | 〃 | 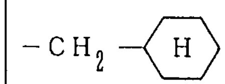 | 0.60-2.00 (22H, m), 3.05 (4H, d, J=6.0Hz), 3.72 (3H, s), 3.78 (6H, s), 5.80 (2H, s) | (184.6) |
| 105 | 〃 | 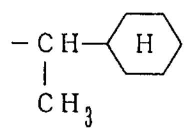 | H | 1.12 (3H, d), 1.25-1.97 (11H, m), 3.00-3.43 (1H, m), 3.75 (3H, s), 3.80 (6H, s), 5.80 (2H, s) | (182.9) |
| 106 | 〃 | 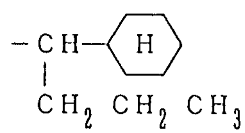 | H | 0.90 (3H, t), 1.03-1.93 (15H, m), 2.87-3.23 (1H, m), 3.73 (3H, s), 3.77 (6H, s), 5.77 (2H, s) | (179.4) |

Table 5 (contd.)

| compound | Ar | $A_1$ | $A_2$ | $^1$H-NMR ($CDCl_3$, δ value)* | m.p. (℃)** |
|---|---|---|---|---|---|
| 107 | 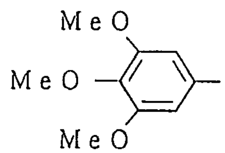 | 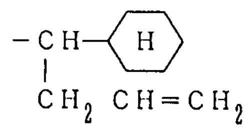 | H | 1.07-1.98 (11H, m), 2.27 (2H, t), 2.98-3.35 (1H, m), 3.72 (3H, s), 3.75 (6H, s), 4.90-5.17 (2H, m), 5.52-6.05 (1H, m), 5.82 (2H, s) | (155.6) |
| 108 | 〃 | 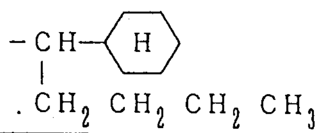 | H | 0.88 (3H, t), 1.03-1.93 (17H, m), 2.90-3.23 (1H, m), 3.72 (3H, s), 3.80 (6H, s), 5.77 (2H, s) | (187.6) |
| 109 | 〃 | 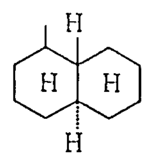 | H | 0.70-2.00 (16H, m), 3.46 (1H, m), 3.72 (3H, s), 3.80 (6H, s), 5.80 (2H, s) | (207.9) |
| 110 | 〃 | 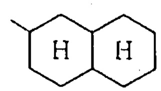 | H | 0.70-2.00 (16H, m), 3.04 (1H, m), 3.70 (3H, s), 3.75 (6H, s), 5.78 (2H, s) | (196.9) |

Table 5 (contd.)

| compound | Ar | $A_1$ | $A_2$ | $^1H$-NMR ($CDCl_3$, δ value‡) | m.p. (℃)‡‡ |
|---|---|---|---|---|---|
| 111 | 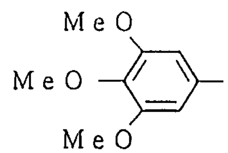 | 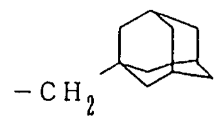 | H | 1.47-2.03 (15H, m), 2.91 (2H, s), 3.77 (3H, s), 3.80 (6H, s), 6.92 (2H, s) ⑤ | (238.1) |
| 112 | 〃 | 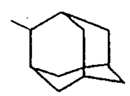 | H | 1.55-2.20 (14H, m), 3.49 (1H, m), 3.72 (3H, s), 3.80 (6H, s), 5.81 (2H, s) | (251.5) |
| 113 | 〃 | $n-C_{12}H_{25}$ | H | 0.85 (3H, t, J=6.0Hz), 1.20-1.80 (20H, m), 3.00 (2H, t, J=6.5Hz), 3.68 (3H, s), 3.74 (6H, s), 3.80 (1H, b), 5.70 (2H, s) | (94-99) |
| 114 | 〃 | 〃 | $n-C_{12}H_{25}$ | 0.87 (6H, t, J=6.0Hz), 1.10-1.65 (40H, m), 2.44 (4H, t, J=7.0Hz), 3.72 (6H, s), 3.80 (3H, s), 5.79 (2H, s) | (68.6) |
| 115 | | 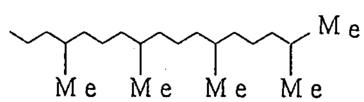 | H | 0.85 (15H, d, J=6.0Hz), 1.05-1.80 (24H, m), 3.06 (2H, t, J=7.0Hz), 3.71 (3H, s), 3.78 (6H, s), 5.79 (2H, s) | Oily |

Table 5 (contd.)

| compound | Ar | $A_1$ | $A_2$ | $^1H$-NMR ($CDCl_3$, δ value)‡ | m.p. (℃)‡‡ |
|---|---|---|---|---|---|
| 116 | 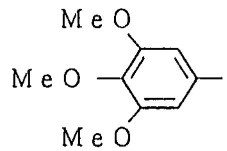 | 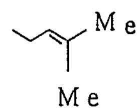 | H | 1.71 (6H, s), 3.12 (1H, b), 3.62 (2H, d, J=7.0Hz), 3.70 (3H, s), 3.76 (6H, s), 5.24 (1H, t, J=7.0Hz), 5.76 (2H, s) | (155) |
| 117 | 〃 | 〃 | 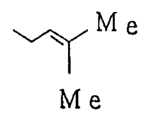 | 1.70 (12H, s), 3.70 (3H, s), 3.75 (6H, s), 3.75-3.90 (4H, m), 5.15 (2H, t, J=6.0Hz), 5.86 (2H, s) | oily |
| 118 | 〃 | 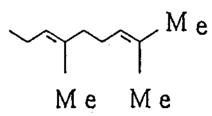 | H | 1.60 (3H, s), 1.70 (6H, s), 2.00-2.18 (4H, m), 2.99 (1H, b), 3.55 (2H, d, J=6.5Hz), 3.72 (3H, s), 3.78 (6H, s), 4.90-5.45 (2H, m), 5.79 (2H, s) | oily |
| 119 | 〃 | 〃 | 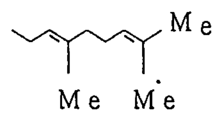 | 1.58 (6H, s), 1.70 (12H, s), 1.95-2.15 (8H, m), 3.73 (3H, s), 3.77 (6H, s), 3.75-3.92 (4H, m), 4.85-5.37 (4H, m), 5.90 (2H, s) | oily |
| 120 | 〃 | 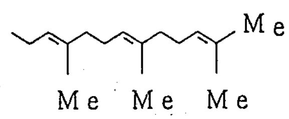 | H | 1.57-1.80 (12H, m), 1.95-2.20 (8H, m), 3.36 (1H, b), 3.65 (2H, d, J=6.5Hz), 3.72 (3H, s), 3.78 (6H, s), 4.88-5.43 (3H, m), 5.78 (2H, s) | oily |

Table 5 (contd.)

| compound | Ar | $A_1$ | $A_2$ | $^1$H-NMR ($CDCl_3$, δ value)‡ | m.p.‡‡ (℃) |
|---|---|---|---|---|---|
| 121 | 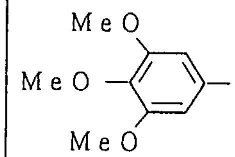 | 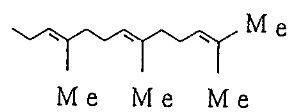 | 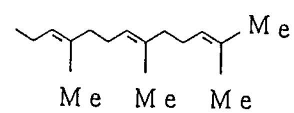 | 1.50-1.70 (24H, m), 1.85-2.10 (16H, m), 3.65 (3H, s), 3.68 (6H, s), 3.65-3.83 (4H, m), 4.72-5.30 (6H, m), 5.80 (2H, s) | oily |
| 122 | 〃 | 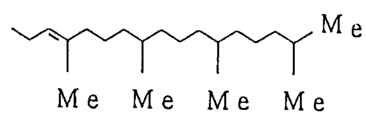 | H | 0.85 (12H, d, J=6.0Hz), 0.70-2.20 (21H, m), 1.68 (3H, s), 3.32-3.80 (3H, m), 3.70 (3H, s), 3.74 (6H, s), 5.24 (1H, t, J=7.5Hz), 5.76 (2H, s) | oily |
| 123 | 〃 | 〃 | Me | 0.85 (12H, d, J=6.0Hz), 1.00-2.20 (21H, m), 1.70 (3H, s), 2.86 (3H, s), 3.75 (3H, s), 3.80 (6H, s), 3.85 (2H, d, J=7.0Hz), 5.20 (1H, t, J=7.0Hz), 5.94 (2H, s) | oily |
| 124 | 〃 | 〃 | 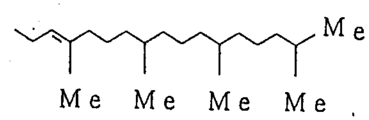 | 0.85 (24H, d, J=6.0Hz), 1.00-2.15 (42H, m), 1.68 (6H, s), 3.70 (3H, s), 3.75 (6H, s), 3.80 (4H, d, J=7.0Hz), 5.15 (2H, t, J=7.0Hz), 5.85 (2H, s) | oily |

Table 5 (contd.)

| compound | Ar | $A_1$ | $A_2$ | $^1$H-NMR ($CDC\ell_3$, δ value‡) | m.p. (°C)‡‡ |
|---|---|---|---|---|---|
| 125 | 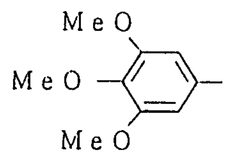 | 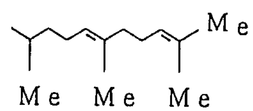 | H | 1.16 (3H, d, J=6.5Hz), 1.55-1.70 (9H, m), 1.90-2.15 (9H, m), 3.38 (1H, m), 3.70 (3H, s), 3.76 (6H, s), 4.00 (1H, b), 4.88-5.25 (2H, m), 5.72 (2H, s) | (122) |
| 126 | 〃 | 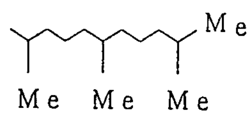 | H | 0.85 (9H, d, J=7.0Hz), 0.80-2.10 (18H, m), 3.33 (2H, b), 3.78 (9H, s), 6.71 (2H, s) ⑤ | (121) |
| 127 | 〃 | 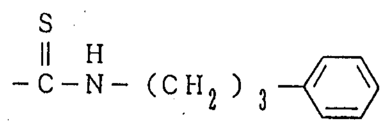 | H | 1.95 (2H, m, J=7.0Hz), 2.63 (2H, t, J=7.0Hz), 3.57 (2H, t, J=7.0Hz), 3.77 (6H, s), 3.80 (3H, s), 5.94 (1H, b), 6.33 (2H, s), 7.10 (5H, s), 7.74 (1H, b) | 138.8 |
| 128 | 〃 | 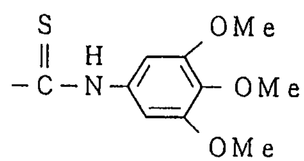 | H | 3.75 (6H, s), 3.80 (12H, s), 6.72 (4H, s), 8.90 (2H, b) ② | 198.2 |

Table 5 (contd.)

| compound | Ar | $A_1$ | $A_2$ | ¹H−NMR ($CDCl_3$, δ value)‡ | m.p. (℃)‡‡ |
|---|---|---|---|---|---|
| 129 | 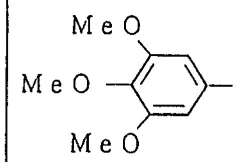 | 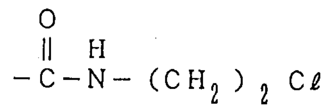 | H | 3.47-3.60 (4H, m), 3.68 (3H, s), 3.76 (6H, s), 6.62 (2H, s) ① | 159 |
| 130 | 〃 | 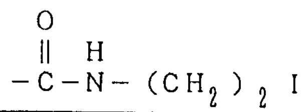 | H | 3.27-3.60 (4H, m), 3.71 (3H, s), 3.78 (6H, s), 6.19 (1H, b), 6.68 (2H, s), 8.19 (1H, b) ② | 155 |
| 131 | 〃 | 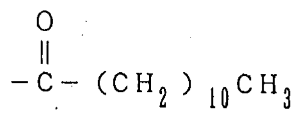 | H | 0.88 (3H, t, J=6.0Hz), 1.10-2.00 (18H, m), 2.31 (2H, t, J=7.0Hz), 3.78 (9H, s), 6.77 (2H, s), 7.35 (1H, b) | 87 |
| 132 | 〃 | 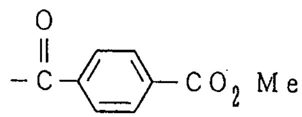 | H | 3.80 (9H, s), 3.90 (3H, s), 6.92 (2H, s), 7.84 (2H, d, J=9.0Hz), 8.06 (2H, d, J=9.0Hz) | 200-201 |
| 133 | 〃 | 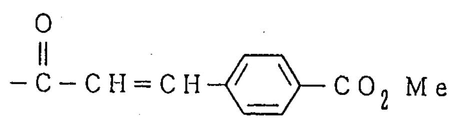 | H | 3.78 (9H, s), 3.87 (3H, s), 6.57 (1H, d, J=16Hz), 6.92 (2H, s), 7.32-8.05 (6H, m) | 201-202 |

Table 5 (contd.)

| compound | Ar | $A_1$ | $A_2$ | $^1$H−NMR ($CDCl_3$, δ value)‡ | m.p. (℃)‡‡ |
|---|---|---|---|---|---|
| 134 | 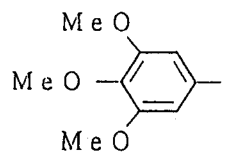 | 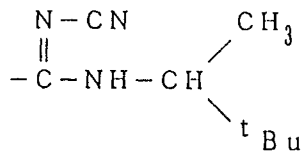 | H | 0.87 (9H, s), 1.05 (3H, d, J=7.0Hz), 3.85 (9H, s), 3.86 (1H, m), 4.80 (1H, d, J=9.0Hz), 6.45 (2H, s), 7.83 (1H, b) | 141-142 |
| 135 | 〃 | 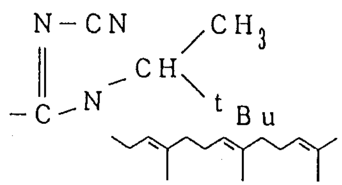 | H | 0.78 (9H, s), 1.05 (3H, d, J=7.0Hz), 1.50-2.15 (20H, m), 3.80 (9H, s), 4.20-5.50 (6H, m), 6.33 (2H, s) | oily |
| 136 | 〃 | $-(CH_2)_2COOMe$ | H | 2.60 (2H, t, J=6.5Hz), 3.40 (2H, t, J=6.5Hz), 3.66 (3H, s), 3.71 (3H, s), 3.77 (6H, s), 4.00 (1H, b), 5.80 (2H, s) | (135) |
| 137 | 〃 | $-(CH_2)_6COOH$ | H | 1.15-1.90 (8H, m), 2.33 (2H, t, J=6.0Hz), 3.04 (2H, t, J=6.0Hz), 3.70 (3H, s), 3.78 (6H, s), 5.75 (2H, b), 5.80 (2H, s) | 76 |

Table 5 (contd.)

| compound | Ar | $A_1$ | $A_2$ | $^1H-NMR$ ($CDCl_3$, δ value)‡ | m.p. (℃)‡‡ |
|---|---|---|---|---|---|
| 138 | 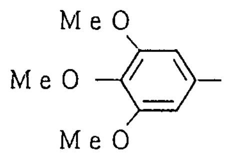 | $-(CH_2)_6COOEt$ | H | 1.22 (3H, t, J=7.0Hz), 1.20-1.90 (8H, m), 2.26 (2H, t, J=7.0Hz), 3.02 (2H, t, J=6.5Hz), 3.48 (1H, b), 3.71 (3H, s), 3.78 (6H, s), 4.06 (2H, q, J=7.0Hz), 5.76 (2H, s) | (110) |
| 139 | 〃 | $-(CH_2)_6COONa$ | $-(CH_2)_6COONa$ | 1.10-1.90 (16H, m), 2.17 (4H, t, J=6.0Hz), 3.20 (4H, t, J=6.5Hz), 3.70 (3H, s), 3.80 (6H, s), 5.80 (2H, s) ① | 185 |
| 140 | 〃 | 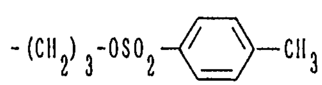 | 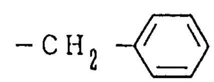 | 1.72-2.15 (2H, m), 2.43 (3H, s), 3.42 (2H, t, J=7.0Hz), 3.72 (9H, s), 4.58 (2H, t, J=5.3Hz), 4.35 (2H, s), 5.88 (2H, s), 7.15-7.75 (9H, m) | oily |
| 141 | 〃 | 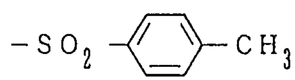 | $-(CH_2)_2CO_2Na$ | 2.38 (3H, s), 2.55 (2H, t, J=7.0Hz), 3.63 (6H, s), 3.70 (2H, t, J=7.0Hz), 3.75 (3H, s), 6.10 (2H, s), 7.12 (2H, d, J=9.0Hz), 7.44 (2H, d, J=9.0Hz) | >200dec |

Table 5 (contd.)

| compound | Ar | $A_1$ | $A_2$ | $^1H$-NMR ($CDCl_3$, δ value)‡ | m.p. (℃)‡‡ |
|---|---|---|---|---|---|
| 142 | 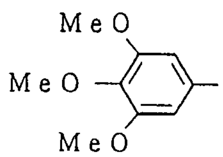 | 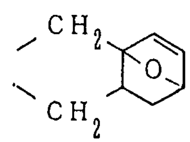 | | No. NMR data (MS:303, 288, 81) | 127.8 |
| 143 | 〃 | 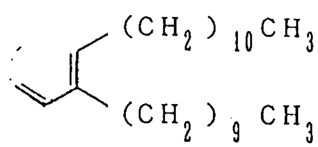 | | 0.87(6H, t, J=6.0Hz), 1.10-2.00(34H, m), 2.55-3.10(4H, m), 3.92(3H, s), 3.95(3H, s), 4.01(3H, s), 7.10(1H, s), 7.18(2H, s), 7.93(1H, s) | oily |
| 144 | 〃 | 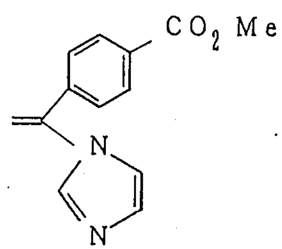 | | 3.62(6H, s), 3.73(3H, s), 3.91(3H, s), 5.92(2H, s), 7.07-8.06(7H, m) | 150 |

Table 5 (contd.)

| compound | Ar | $A_1$ | $A_2$ | $^1$H-NMR ($CDCl_3$, δvalue)* | m.p.** (℃) |
|---|---|---|---|---|---|
| 145 | 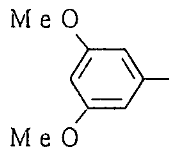 | 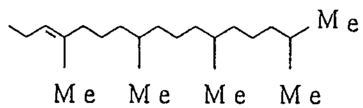 | H | 0.85 (12H, d, J=6.0Hz), 1.00-2.20 (21H, m), 1.67 (3H, s), 3.64 (2H, d, J=7.0Hz), 3.70 (6H, s), 5.27 (1H, t, J=7.0 Hz), 5.75 (3H, m) | oily |
| 146 | 〃 | 〃 | 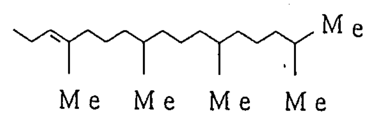 | 0.85 (24H, d, J=6.0Hz), 1.00-2.20 (42H, m), 1.67 (6H, s), 3.72 (6H, s), 3.83 (4H, d, J=7.0Hz), 5.20 (2H, t, J=7.0Hz), 5.86 (3H, s) | oily |
| 147 | 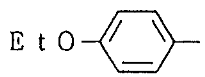 | 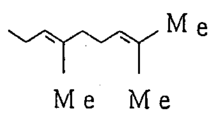 | H | 1.31 (3H, t, J=7.0Hz), 1.57 (3H, s), 1.64 (6H, s), 1.93-2.20 (4H, m), 3.10 (1H, b), 3.57 (2H, d, J=7.0Hz), 3.87 (2H, q, J=7.0Hz), 4.85-5.40 (2H, m), 6.41 (2H, d, J=9.0Hz), 6.67 (2H, d, J=9.0Hz) | oily |

Table 5 (contd.)

| compound | Ar | $A_1$ | $A_2$ | $^1H$-NMR ($CDCl_3$, δ value)‡ | m.p.‡‡ (℃) |
|---|---|---|---|---|---|
| 148 | 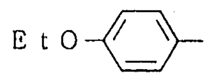 | 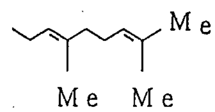 | 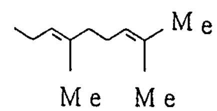 | 1.33 (3H, t, J=7.0Hz), 1.57 (6H, s), 1.63 (12H, s), 1.93-2.12 (8H, m), 3.70 (4H, d, J=7.0Hz), 3.89 (2H, q, J=7.0Hz), 4.80-5.30 (4H, m), 6.63 (4H, s) | oily |
| 149 | 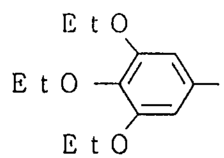 | 〃 | H | 1.32 (3H, t, J=7.0Hz), 1.40 (6H, t, J=7.0Hz), 1.53 (6H, s), 1.70 (3H, s), 1.85-2.20 (4H, m), 3.45 (1H, b), 3.66 (2H, d, J=7.0Hz), 3.94 (2H, q, J=7.0Hz), 4.00 (4H, q, J=7.0Hz), 5.30 (2H, t, J=7.0Hz), 5.87 (2H, s) | oily |
| 150 | 〃 | 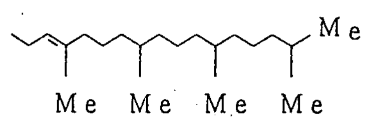 | H | 0.85 (12H, d, J=6.0Hz), 1.03-2.20 (21H, m), 1.30 (3H, t, J=7.0Hz), 1.37 (6H, t, J=7.0Hz), 1.68 (3H, s), 3.61 (2H, d, J=7.0Hz), 3.91 (2H, q, J=7.0Hz), 3.98 (4H, q, J=7.0Hz), 5.26 (1H, t, J=7.0Hz), 5.80 (2H, s) | (69) |

Table 5 (contd.)

| compound | Ar | $A_1$ | $A_2$ | $^1H-NMR$ ($CDCl_3$, δ value)* | m.p.** (℃) |
|---|---|---|---|---|---|
| 151 | 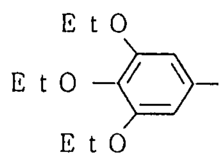 | $-CO_2{}^tBu$ | 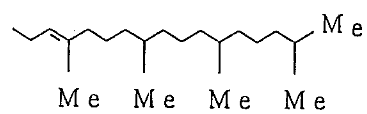 | 0. 86 (12H, d, J=0. 6Hz), 1. 00-2. 15 (21H, m), 1. 38 (9H, t, J=7. 0Hz), 1. 42 (9H, s), 1. 50 (3H, s), 4. 00 (6H, q, J=7. 0Hz), 5. 25 (1H, t, J=7. 0Hz), 6. 35 (2H, s) | oily |
| 152 | 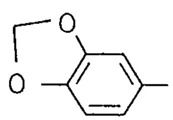 | 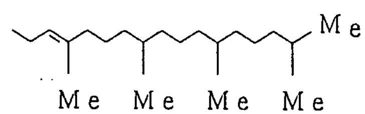 | H | 0. 85 (12H, d, J=6. 0Hz), 1. 00-2. 20 (21H, m), 1. 67 (3H, s), 3. 35 (1H, b), 3. 61 (2H, d, J=7. 0Hz), 5. 30 (1H, t, J=7. 0Hz), 5. 80 (2H, s), 5. 90-6. 70 (3H, m) | oily |
| 153 | 〃 | 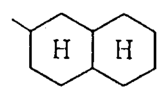 | H | 0. 70-2. 20 (16H, m), 3. 10 (1H, m), 5. 80 (2H, s), 6. 00 (1H, dd, J=9. 0Hz, 3. 0Hz), 6. 20 (1H, d, J=3. 0Hz), 6. 60 (1H, d, J=9. 0Hz) | (191dec) |

Table 5 (contd.)

| compound | Ar | $A_1$ | $A_2$ | ¹H-NMR ($CDCl_3$, δ value)* | m.p. (℃)** |
|---|---|---|---|---|---|
| 154 | 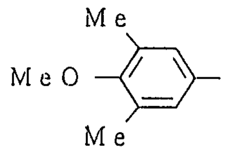 | 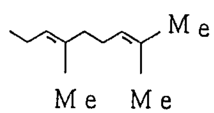 | H | 1.57-1.78 (9H, m), 1.95-2.17 (4H, m), 2.23 (6H, s), 3.63 (3H, s), 3.67 (2H, d), 4.98-5.42 (2H, m), 6.22 (2H, s) | (87.1) |
| 155 | 〃 | 〃 | 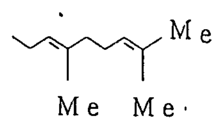 | 1.52-1.77 (18H, m), 1.93-2.13 (8H, m), 2.20 (6H, s), 3.60 (3H, s), 3.75 (4H, d), 4.90-5.30 (4H, m), 6.30 (2H, s) | oily |
| 156 | 〃 | 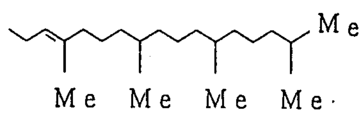 | H | 0.85 (12H, d), 1.03-2.17 (21H, m), 1.70 (3H, s), 2.23 (6H, s), 3.65 (2H, d, J=6.0Hz), 3.67 (3H, s), 5.32 (1H, t, J=6.0Hz), 6.28 (2H, s) | oily |
| 157 | 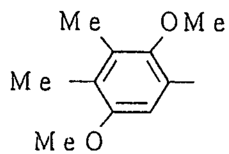 | $n-C_{12}H_{25}$ | H | 0.87 (3H, t, J=6.0Hz), 1.10-1.80 (20H, m), 2.01 (3H, s), 2.13 (3H, s), 3.06 (2H, t, J=7.0Hz), 3.60 (3H, s), 3.72 (3H, s), 4.02 (1H, b), 6.03 (1H, s) | oily |

Table 5 (contd.)

| compound | Ar | $A_1$ | $A_2$ | $^1$H-NMR ($CDCl_3$, δ value)* | m.p. (℃)** |
|---|---|---|---|---|---|
| 158 | 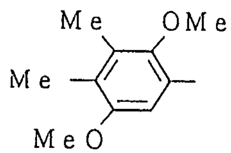 | 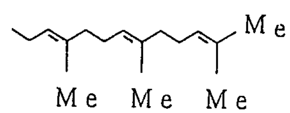 | H | 1.55-1.78 (12H, m), 1.95-2.20 (8H, m), 2.04 (3H, s), 2.15 (3H, s), 3.60 (3H, s), 3.73 (3H, s), 3.60-4.00 (3H, m), 4.90-5.50 (3H, m), 6.10 (1H, s) | oily |
| 159 | 〃 | 〃 | 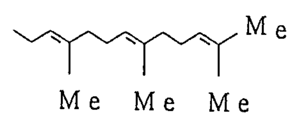 | 1.55-1.75 (24H, m), 1.90-2.20 (16H, m), 2.04 (3H, s), 2.14 (3H, s), 3.67 (4H, d, J=7.0Hz), 3.69 (6H, s), 4.70-5.35 (6H, m), 6.23 (1H, s) | oily |
| 160 | 〃 | $-COCH_2Cl$ | 〃 | 0.86 (3H, t, J=6.0Hz), 1.10-1.80 (20H, m), 2.12 (3H, s), 2.19 (3H, s), 3.59 (3H, s), 3.66 (2H, m), 3.73 (3H, s), 3.85 (2H, s), 6.38 (1H, s) | oily |

Table 5 (contd.)

| compound | Ar | $A_1$ | $A_2$ | $^1H-NMR$ ($CDCl_3$, $\delta$ value)‡ | m.p.‡‡ (℃) |
|---|---|---|---|---|---|
| 161 | 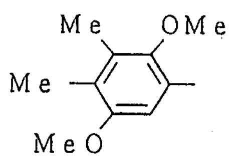 | $-C(=O)CH_2S(CH_2)_{11}CH_3$ | H | 0.87 (3H, t, J=6.0Hz), 1.10-1.80 (20H, m), 2.10 (3H, s), 2.20 (3H, s), 2.60 (2H, t, J=7.0Hz), 3.36 (2H, s), 3.70 (3H, s), 3.80 (3H, s), 7.80 (1H, s), 9.20 (1H, b) | 58 |
| 162 | 〃 | 〃 | Me | 0.87 (3H, t, J=6.0Hz), 1.10-1.80 (20H, m), 2.13 (3H, s), 2.21 (3H, s), 2.57 (2H, m), 3.26 (2H, s), 3.31 (3H, s), 3.60 (3H, s), 3.75 (3H, s), 6.35 (1H, b) | oily |
| 163 | 〃 | 〃 | 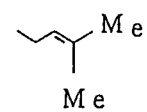 | 0.87 (3H, t, J=6.0Hz), 1.10-1.45 (20H, m), 1.50 (3H, s), 1.67 (3H, s), 2.10 (3H, s), 2.18 (3H, s), 2.60 (2H, t, J=7.0Hz), 3.07 (2H, s), 3.61 (3H, s), 3.70 (3H, s), 3.85-4.90 (2H, m), 5.30 (1H, t, J=7.0Hz), 6.53 (1H, s) | oily |

Table 5 (contd.)

| compound | Ar | $A_1$ | $A_2$ | $^1H-NMR$ ($CDCl_3$, δ value)‡ | m.p.‡‡ (℃) |
|---|---|---|---|---|---|
| 164 | 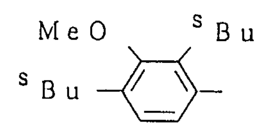 | 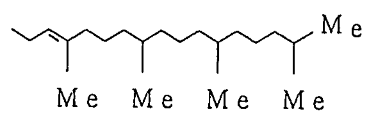 | H | 0.72-2.03 (37H, m), 0.87 (12H, d), 1.72 (3H, s), 2.20-2.67 (1H, m), 2.75-3.20 (1H, m), 3.67 (3H, s), 4.00 (2H, d, J=6.0Hz), 5.33 (1H, t, J=6.0Hz), 6.22-6.35 (2H, m) | oily |
| 165 | 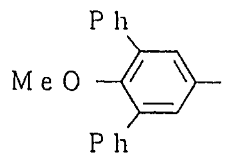 | 〃 | H | 0.87 (12H, d), 1.02-2.17 (21H, m), 1.68 (3H, s), 3.05 (3H, s), 3.72 (2H, d, J=6.0Hz), 5.33 (1H, t, J=6.0Hz), 6.55 (2H, s), 7.27-7.67 (10H, m) | (104) |
| 166 | 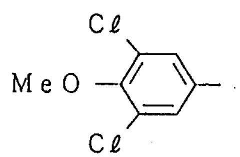 | 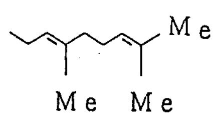 | H | 1.53-1.72 (9H, m), 2.00-2.15 (4H, m), 3.58 (2H, d), 3.75 (3H, s), 4.85-5.32 (2H, m), 6.42 (2H, s) | (83) |
| 167 | 〃 | 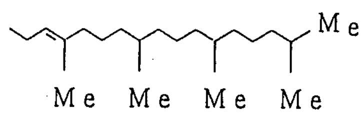 | H | 0.85 (12H, d), 1.00-2.17 (21H, m), 1.67 (3H, s), 3.57 (2H, d, J=6.0Hz), 3.75 (3H, s), 5.18 (1H, t, J=6.0Hz), 6.40 (2H, s) | (64) |

Table 5 (contd.)

| compound | Ar | $A_1$ | $A_2$ | $^1H-NMR$ ($CDCl_3$, δ value)‡ | m.p. (℃)‡‡ |
|---|---|---|---|---|---|
| 168 | 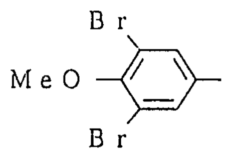 | 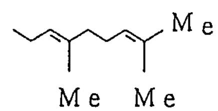 | H | 1.48-1.67 (9H, m), 1.87-2.13 (4H, m), 3.87 (3H, s), 3.90 (2H, d), 4.95-5.57 (2H, m), 7.77 (2H, s) | (72) |
| 169 | 〃 | 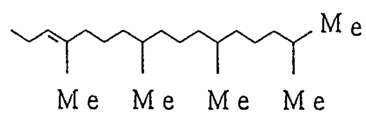 | H | 0.87 (12H, d), 1.02-2.18 (21H, m), 1.70 (3H, s), 3.60 (2H, d, J=6.0Hz), 3.78 (3H, s), 5.22 (1H, t, J=6.0Hz), 6.67 (2H, s) | oily |
| 170 | 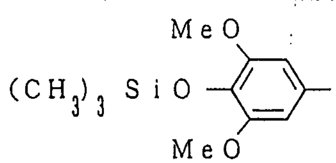 | $-CH_2-$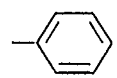 | H | 0.00 (9H, s), 3.50 (6H, s), 4.33 (2H, s), 6.46 (2H, s), 7.19 (5H, s) | 140 |
| 171 | 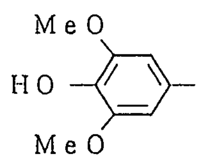 | $-COCF_3$ | $-CH_2-$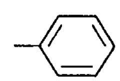 | 3.68 (6H, s), 4.81 (2H, s), 5.51 (1H, s), 6.10 (2H, s), 7.20 (5H, s) | 108 |

Table 5 (contd.)

| compound | Ar | $A_1$ | $A_2$ | $^1$H-NMR ($CDCl_3$, δ value) | m.p. (℃) ** |
|---|---|---|---|---|---|
| 172 | 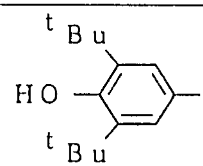 | $-CH_2$ 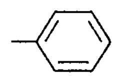 | H | No NMR data (MS: 311, 309, 294, 220, 91) | [168dec] |
| 173 | 〃 | $-COCH_3$ | $-CH_2$ 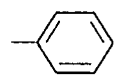 | 1.31 (18H, s), 1.83 (3H, s), 4.75 (2H, s), 5.15 (1H, s), 6.59 (2H, s), 7.14 (5H, s) | 154 |
| 174 | 〃 | 〃 | $-(CH_2)_7CH_3$ | 0.86 (3H, t, J=6.0Hz), 1.15-1.40 (12H, m), 1.42 (18H, s), 1.78 (3H, s), 3.60 (2H, t, J=7.0Hz), 5.25 (1H, s), 6.87 (2H, s) | 116 |
| 175 | 〃 | $-CO$ 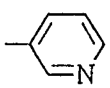 | $-CH_2$ 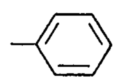 | 1.20 (18H, s), 5.06 (2H, s), 5.15 (1H, s), 6.68 (2H, s), 6.95-8.47 (4H, m), 7.30 (5H, s) | 151 |
| 176 | 〃 | $-CO$ 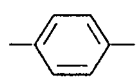 $CO_2Me$ | $-CH_2$ 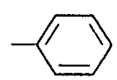 | 1.20 (18H, s), 3.84 (3H, s), 5.05 (3H, s), 6.53 (2H, s), 7.27 (2H, d, J=9.0Hz), 7.29 (5H, s), 7.80 (2H, d, J=9.0Hz) | 149-150 |

Table 5 (contd.)

| compound | Ar | $A_1$ | $A_2$ | $^1$H−NMR ($CDCl_3$, δ value) | m.p. (℃) ‡‡ |
|---|---|---|---|---|---|
| 177 | 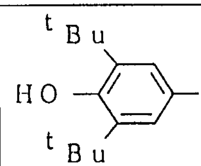 | 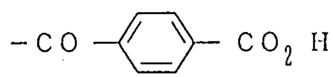 | 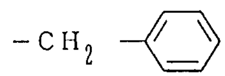 | 1.17 (18H, s), 5.05 (3H, s), 6.49 (1H, b), 6.53 (2H, s), 7.28 (5H, s), 7.28 (2H, d, J=9.0Hz), 7.83 (2H, d, J=9.0Hz) | 202-203 |
| 178 | 〃 | 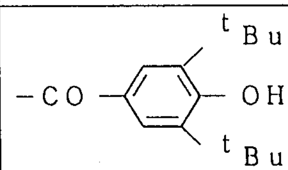 | 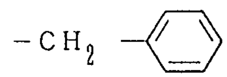 | 1.18 (9H, s), 1.26 (18H, s), 1.49 (9H, s), 5.10 (1H, s), 5.12 (2H, s), 5.75 (1H, s), 6.66 (2H, s), 7.33 (5H, s), 8.02 (2H, s) | 233-234 |
| 179 | 〃 | 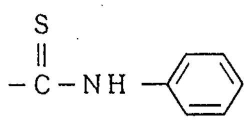 | 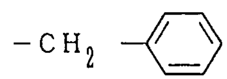 | 1.33 (18H, s), 5.33 (1H, s), 5.52 (2H, s), 6.78 (2H, s), 7.12-7.50 (10H, m) | 173 |
| 180 | 〃 | 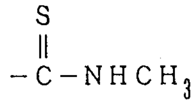 | 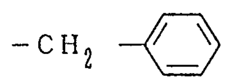 | 1.29 (18H, s), 3.05 (3H, d, J=5.0Hz), 5.26 (1H, s), 5.39 (2H, s), 5.43 (1H, b), 6.60 (2H, s), 7.20 (5H, s) | 160-161 |
| 181 | 〃 | $-(CH_2)_7CH_3$ | H | 0.86 (3H, t, J=6.0Hz), 1.10-1.40 (12H, m), 1.40 (18H, s), 3.20 (2H, t, J=7.0Hz), 7.28 (2H, s), 8.22 (4H, b) ②⑥ | [162-164] |

Table 5 (contd.)

| compound | Ar | $A_1$ | $A_2$ | $^1$H-NMR ($CDCl_3$, δ value) | m.p. (℃) ‡‡ |
|---|---|---|---|---|---|
| 182 | 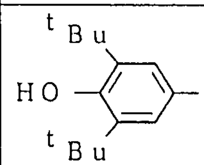 | $-CO_2Et$ | $-CH_2$ 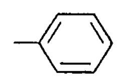 | 1.21 (3H, t, J=7.0Hz), 1.33 (18H, s), 4.19 (2H, q, J=7.0Hz), 4.75 (2H, s), 5.10 (1H, s), 6.80 (2H, s), 7.23 (5H, s) | 104 |
| 183 | 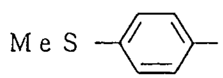 | 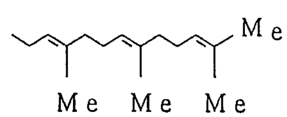 | H | 1.53-1.75 (12H, m), 1.92-2.18 (8H, m), 2.36 (3H, s), 3.33 (1H, b), 3.63 (2H, d, J=7.0Hz), 4.85-5.42 (3H, m), 6.44 (2H, d, J=8.5Hz), 7.12 (2H, d, J=8.5Hz) | oily |

$Ar-A_3-A_4$

| compound | Ar | $A_3$ | $A_4$ | $^1$H-NMR ($CDCl_3$, δ value) | m.p. (℃) ‡‡ |
|---|---|---|---|---|---|
| 184 | 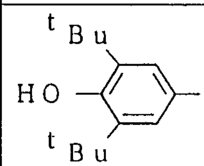 | $-CH_2-$ | $-NHC_2H_5$ | 1.12 (3H, t, J=7.0Hz), 1.43 (18H, s), 2.70 (2H, q, J=7.0Hz), 3.68 (2H, s), 5.10 (1H, br), 7.09 (2H, s) | (224) |
| 185 | 〃 | 〃 | $-NHCH_2$ 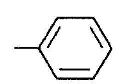 | 1.46 (18H, s), 1.70 (1H, br), 3.72 (2H, s), 3.84 (2H, s), 5.13 (1H, br), 7.15 (2H, s), 7.34 (5H, s) | (112-113) |

Table 5 (contd.)

| compound | Ar | $A_3$ | $A_4$ | $^1$H-NMR ($CDCl_3$, δ value)‡ | m.p. (℃)‡‡ |
|---|---|---|---|---|---|
| 186 | 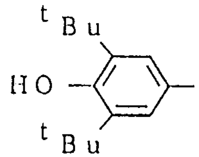 | $-CH_2-$ | 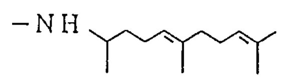 | 1.10 (3H, d, J=7.0Hz), 1.42 (18H, s), 1.60-1.72 (9H, m), 1.95-2.15 (8H, m), 2.72 (1H, m), 3.66 (2H, s), 5.09 (2H, m), 7.05 (2H, s) | [210-211] |
| 187 | 〃 | -S- | 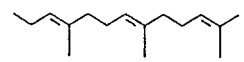 | 1.40 (18H, s), 1.55-1.70 (12H, m), 1.85-2.10 (8H, m), 3.40 (2H, d, J=7.0Hz), 4.60-5.50 (3H, m), 5.12 (1H, s), 7.20 (2H, s) | oily |
| 188 | 〃 | 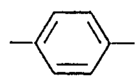 | 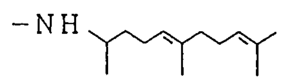 | 1.21 (3H, d, J=7.0Hz), 1.50 (18H, s), 1.55-2.40 (17H, m), 3.52 (1H, m), 4.95-5.36 (3H, m), 6.62 (2H, d, J=9.0Hz), 7.34 (2H, s), 7.38 (2H, d, J=9.0Hz) | (85-86) |
| 189 | 〃 | 〃 | 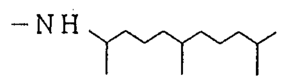 | 0.87 (9H, d, J=7.0Hz), 1.05-1.70 (17H, m), 1.50 (18H, s), 3.50 (1H, m), 5.12 (1H, s), 6.62 (2H, d, J=9.0Hz), 7.34 (2H, s), 7.38 (2H, d, J=9.0Hz) | [142] |

Table 5 (contd.)

| compound | Ar | $A_3$ | $A_4$ | $^1$H-NMR ($CDCl_3$, δ value) | m.p. (°C) ** |
|---|---|---|---|---|---|
| 190 | 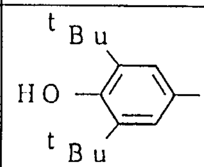 | 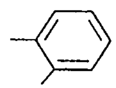 | 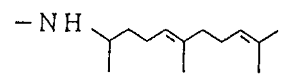 | 1.12 (3H, d, J=7.0Hz), 1.45 (18H, s), 1.50-1.70 (9H, m), 1.94-2.14 (8H, m), 3.46 (1H, m), 3.80 (1H, br), 5.08 (2H, m), 5.18 (1H, s), 6.55-7.22 (4H, m), 7.18 (2H, s) | (94-95) |
| 191 | 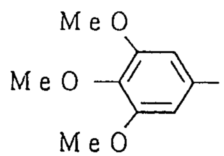 | -S- | 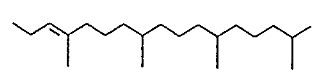 | 0.85 (12H, d, J=6.0Hz), 1.00-2.10 (21H, m), 1.55 (3H, s), 3.50 (2H, d, J=7.0Hz), 3.78 (9H, s), 5.25 (1H, m), 6.53 (2H, s) | oily. |

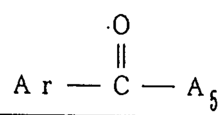

| compound | Ar | $A_5$ | $^1$H-NMR ($CDCl_3$, δ value) | m.p. (°C) ** |
|---|---|---|---|---|
| 192 | 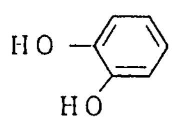 | 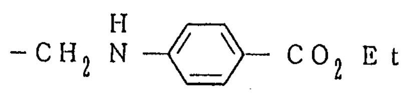 | 1.42 (3H, t, J=7.0Hz), 3.32 (2H, q, J=7.0Hz), 4.25 (2H, s), 4.8-5.8 (3H, br), 6.7-7.8 (6H, m) ② | 180-193 |

Table 5 (contd.)

| compound | Ar | $A_5$ | $^1H$-NMR ($CDCl_3$, δ value) | m.p. (°C) |
|---|---|---|---|---|
| 193 | 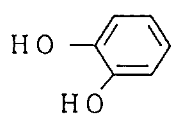 | 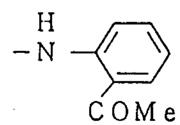 | 2.8 (3H, s), 3.2-3.9 (2H, br), 6.9-8.3 (7H, m), 8.8-9.1 (1H, m) ② | 190-192 |
| 194 | 〃 | 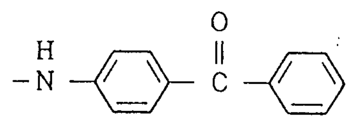 | 3.1-3.9 (2H, br), 6.8-8.2 (13H, m) ② | 230-236 |
| 195 | 〃 | 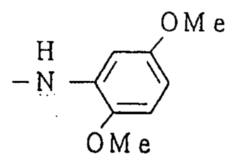 | 3.0-3.7 (3H, br), 3.80 (3H, s), 3.92 (3H, s), 6.5-7.5 (4H, m), 7.9-8.2 (1H, m), 8.7 (1H, brs) ② | 170-172 |
| 196 | 〃 | 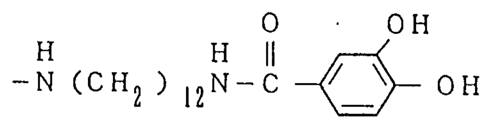 | 1.3-2.0 (20H, br), 3.2-3.6 (4H, br), 5.3-6.1 (6H, br), 6.8-7.5 (4H, m), 8.3 (2H, brs) ② | 211-214 |

Table 5 (contd.)

| compound | Ar | $A_5$ | $^1H-NMR$ ($CDCl_3$, δ value) | m.p. (℃) ** |
|---|---|---|---|---|
| 197 | 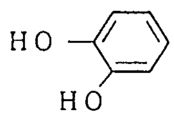 | 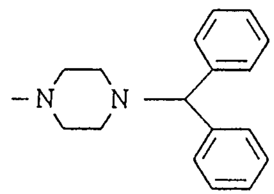 | 2.2-2.7 (4H, m), 3.0-3.8 (2H, br), 3.4-3.8 (4H, m), 4.30 (1H, s), 6.7-7.6 (15H, m) ② | 235-239 |
| 198 | 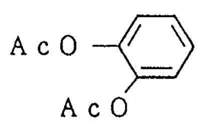 | 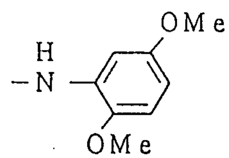 | 2.17 (6H, s), 3.76 (3H, s), 3.88 (3H, s), 6.4-9.0 (7H, m) ② | 150-158 |
| 199 | 〃 | 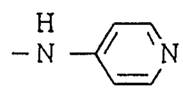 | 2.35 (6H, s), 6.7-8.5 (7H, m) ② | 270-274 |
| 200 | 〃 | 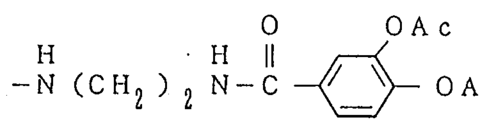 | 2.30 (12H, s), 3.6 (4H, brs), 4.3-4.7 (2H, br), 6.7-7.8 (6H, m) ② | 251-257 |

Table 5 (contd.)

| compound | Ar | $A_5$ | $^1$H-NMR ($CDCl_3$, δ value)‡ | m.p. (℃) ‡‡ |
|---|---|---|---|---|
| 201 | 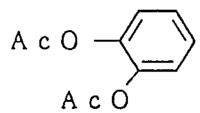 | 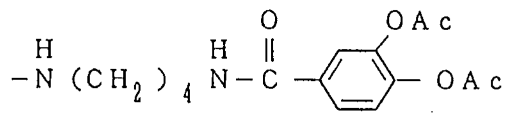 | 1.40 (4H, q, J=7.0Hz), 2.30 (12H, s), 3.25 (4H, q, J=7.0Hz), 6.67-8.0 (6H, m) ② | 123-140 |
| 202 | 〃 | 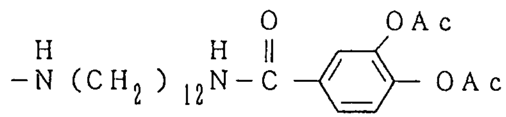 | 1.2-1.9 (20H, br), 2.36 (12H, s), 3.46 (4H, brq), 6.0-6.4 (2H, br), 7.2-7.8 (6H, m) ② | 141-143 |
| 203 | 〃 | 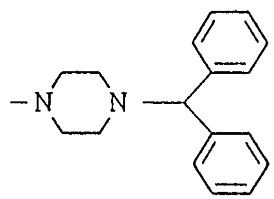 | 2.3-2.7 (4H, m), 3.5-3.8 (4H, m), 4.35 (1H, s), 6.7-7.7 (13H, m) ② | 105-111 |

Table 5 (contd.)

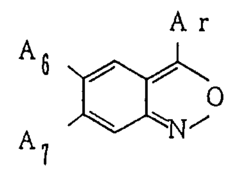

| compound | Ar | $A_6$ | $A_7$ | $^1$H-NMR ($CDCl_3$, δ value)‡ | m.p. (℃)‡‡ |
|---|---|---|---|---|---|
| 204 | 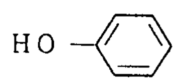 | H | Cℓ | 3.0-3.4 (1H, br), 6.6-7.6 (7H, m) ② | 157-163 |
| 205 | 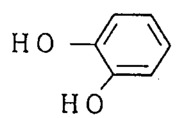 | Cℓ | H | 3.92 (2H, brs), 6.7-7.3 (3H, m), 7.86 (3H, brs) ② | 187-190 |

Table 5 (contd.)

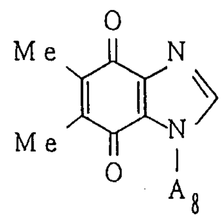

| compound | $A_8$ | ¹H-NMR ($CDCl_3$, δ value)‡ | m.p. (℃)‡‡ |
|---|---|---|---|
| 206 | 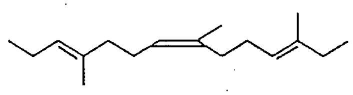 | 1.58-1.70 (9H, m), 1.82 (3H, s), 2.00-2.25 (8H, m), 2.10 (6H, s), 4.90-5.66 (5H, m), 8.21 (1H, s) | oily |
| 207 | 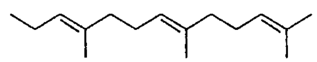 | 1.61-1.77 (9H, m), 1.87 (3H, s), 2.05-2.20 (8H, m), 2.10 (6H, s), 4.90-5.60 (5H, m), 7.90 (1H, s) | oily |

Table 5 (contd.)

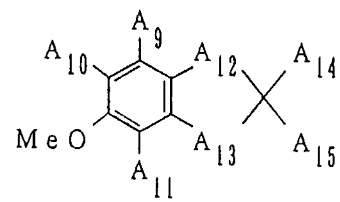

| compound | $A_{9-11}$ | $A_{12}$ | $A_{13}$ | $A_{14}$, $A_{15}$ | $^1H-NMR$ ($CDCl_3$, δ value)‡ | m.p. (℃)‡‡ |
|---|---|---|---|---|---|---|
| 208 | $A_9$ : MeO<br>$A_{10}$ : OH<br>$A_{11}$ : H | 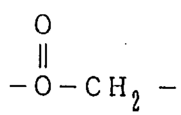 | −N−<br>H | $A_{14}$ : Me<br>$A_{15}$ : H | 1. 30 (3H, d, J=7. 0Hz), 2. 36-2. 60 (2H, m), 3. 62 (1H, m), 3. 73 (3H, s), 3. 77 (3H, s), 4. 44 (1H, br), 5. 60 (1H, s), 12. 45 (1H, s) | 120 |
| 209 | 〃 | 〃 | 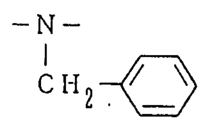 | 〃 | 1. 23 (3H, d, J=7. 0Hz), 2. 40 (1H, dd, J=4. 0, 17. 0Hz), 3. 07 (1H, dd, J=6. 0, 17. 0Hz), 3. 61 (3H, s), 3. 70 (3H, s), 3. 60-4. 20 (1H, m), 4. 40 (2H, d, J=7. 0Hz), 5. 45 (1H, s), 7. 25 (5H, s) | (115) |

Table 5 (contd.)

| compound | $A_{9-11}$ | $A_{12}$ | $A_{13}$ | $A_{14}$, $A_{15}$ | ¹H−NMR ($CDCl_3$, δ value)* | m.p. (℃)** |
|---|---|---|---|---|---|---|
| 210 | $A_9$ : MeO<br>$A_{10}$ : MeO<br>$A_{11}$ : H | O<br>‖<br>$-C-CH_2-$ | −N−<br>H | $A_{14}$ : Me<br>$A_{15}$ : H | 1.29 (3H, d, J=7.0Hz), 2.32−2.55 (2H, m), 3.60 (1H, m), 4.52 (1H, br), 5.90 (1H, s) | 148 |
| 211 | 〃 | OH<br>\|<br>$-C-CH_2-$<br>\|<br>H | 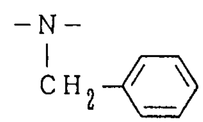 | $A_{14}$ : H<br>$A_{15}$ : H | 1.80−3.50 (5H, m), 3.58 (3H, s), 3.70 (3H, s), 3.93 (3H, s), 4.42 (2H, s), 4.95 (1H, br), 5.89 (1H, s), 7.25 (5H, s) | oily |
| 212 | 〃 | Me<br>\|<br>$-C-CH_2-$<br>\|<br>H | −N−<br>H | $A_{14}$ : Me<br>$A_{15}$ : Me | 1.10 (3H, s), 1.20 (3H, s), 1.35 (3H, d, J=7.0Hz), 1.50−2.00 (2H, m), 3.00 (1H, m), 3.42 (1H, br), 3.70 (3H, s), 3.75 (3H, s), 3.85 (3H, s), 5.82 (1H, s) | (168-169) |
| 213 | 〃 | Me<br>\|<br>$-C=CH-$ | 〃 | 〃 | 1.20 (6H, s), 2.14 (3H, s), 3.77 (6H, s), 3.80 (3H, s), 5.17 (1H, br), 5.85 (1H, s) | (116-119) |

Table 5 (contd.)

| compound | $A_{9-11}$ | $A_{12}$ | $A_{13}$ | $A_{14}$, $A_{15}$ | $^1H-NMR$ ($CDCl_3$, δ value)‡ | m.p. (℃) ‡‡ |
|---|---|---|---|---|---|---|
| 214 | $A_9$ : MeO<br>$A_{10}$ : MeO<br>$A_{11}$ : H | $-CH_2CH_2-$ | 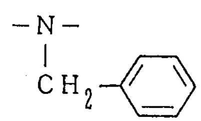 | $A_{14}$ : Me<br>$A_{15}$ : H | 1.13 (3H, d, J=7.0Hz), 1.70-2.05 (2H, m), 2.55-2.88 (2H, m), 3.30-3.65 (1H, m), 3.52 (3H, s), 3.72 (3H, s), 3.82 (3H, s), 4.40 (2H, s), 5.78 (1H, s), 7.22 (5H, s) | oily |
| 215 | $A_9$ : H<br>$A_{10}$ : MeO<br>$A_{11}$ : MeO | 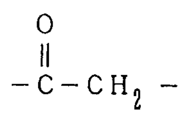 | $-O-$ | $A_{14}$ : 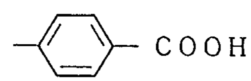<br>$A_{15}$ : H | 3.93 (3H, s), 4.00 (3H, s), 4.09 (3H, s), 6.99 (1H, s), 7.30 (1H, s), 8.12 (4H, s) ④ | >250 |

* With respect to the data of $^1$H-NMR:

those expressed in ① were measured by using $CDCl_3$ + $CD_3OD$;

those expressed in ② were measured by using $CDCl_3$ + DMSO-$d_6$;

those expressed in ③ were measured by using $CD_3OD$ + DMSO-$d_6$;

those expressed in ④ were measured by using DMSO-$d_6$;

those expressed in ⑤ were measured in the form of hydrochloride;

those expressed in ⑥ were measured in the form of oxalate; and

those having no mark were measured by using $CDCl_3$ in a free state.

** With respect to the data of m.p.:

those given in (    ) were measured as hydrochloride;

those given in <    > were measured as fumarate;

those given in [    ] were measured as oxalate; and

those having no mark were measured as a free state.

## INDUSTRIAL APPLICABILITY

The compound of the present invention has an effect of suppressing the negative charge of LDL and thus suppresses the denaturation of LDL required in the recognition of LDL by scavenger receptors. Accordingly it is available as a drug, more particularly, as a treatment for arteriosclerosis, peptic ulcers, cancer, ischemic organopathy, inflammation and pulmonary diseases caused by, for example, silicon dust.

## Claims

**1.** A drug composition which comprises a compound suppressing the negative charge of LDL and pharmaceutically acceptable carrier(s).

**2.** A drug composition as claimed in Claim 1, wherein the negative charge of LDL is confirmed by agarose gel electrophoresis and/or the TBARS level due to the oxidation of LDL with $Cu^{2+}$.

**3.** A drug composition as claimed in Claim 1 or 2 which is a remedy for arteriosclerosis.

**4.** A drug composition as claimed in Claim 1 or 2 which is a treatment for peptic ulcers, cancer, ischemic organopathy, inflammation and pulmonary diseases caused by, for example, silicon dust.

**5.** A drug composition as claimed in each of Claims 1 to 4 which is a compound represented by the following general formulae (I) to (VI):
a compound represented by the general formula (I):

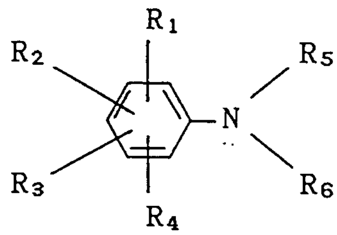

(I)

wherein $R_1$, $R_2$, $R_3$ and $R_4$ are each selected from a group consisting of a hydrogen atom, a hydroxy group, an optionally branched alkyl group having 1 to 5 carbon atoms, an alkoxy group having 1 to 5 carbon atoms, a methylthio group, a trimethylsilyloxy group, a methylenedioxy group, a halogen atom and a phenyl group;

$R_5$ is selected from a group consisting of a group represented by the following general formula (I)-1:

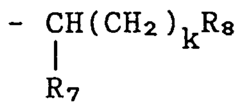

(I) - 1

wherein $R_7$ is selected from a group consisting of a hydrogen atom, an alkyl group having 1 to 5 carbon atoms, an alkenyl group having 1 to 5 carbon atoms, a phenyl group and a cyano group;

k is an integer of from 0 to 8; and

$R_8$ is selected from a group consisting of an option-ally branched alkyl group having 1 to 20 carbon atoms, an optionally branched alkenyl group having 1 to 20 carbon atoms optionally substituted with a phenyl group, an optionally substituted phenyl group, an optionally substituted heterocyclic group, a cycloalkyl group having 3 to 8 carbon atoms, a naphthyl group, an adamantyl group, a tosyloxy group, a hydroxy group and a group represented by the following general formula:

$CO_2R_9$

wherein $R_9$ is selected from a group consisting of a hydrogen atom and an alkyl group having 1 to 5 carbon atoms;

a group represented by the following general formula (I)-2:

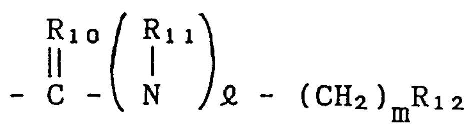

(I) - 2

wherein $R_{10}$ is selected from a group consisting of O, S and NCN;

$R_{11}$ represents a hydrogen atom or an optionally branched alkenyl group having 1 to 20 carbon atoms;

$\ell$ is an integer of 0 or 1;

m is an integer of from 0 to 10; and

$R_{12}$ is selected from a group consisting of an optionally branched alkyl group having 1 to 10 carbon atoms, an alkenyl group having 1 to 5 carbon atoms optionally substituted with a phenyl group, an alkoxy group having 1 to 5 carbon atoms, an optionally substituted phenyl group, a trifluoromethyl group, an alkylthio group having 1 to 20 carbon atoms, a halogen atom, a pyridyl group and a chloromethyl group;

a decalyl group, a tetralyl group, an adamantyl group, a tosyl group and a chromanyl group; and

$R_6$ is selected from a group consisting of a hydrogen atom, an alkyl group having 1 to 20 carbon atoms, a group represented by the following general formula (I)-3:

$-(CH_2)_n R_{13}$ (I) - 3

wherein n is an integer of from 1 to 6; and

$R_{13}$ is selected from a group consisting of a hydroxy group, an optionally substituted phenyl group, a cyclohexyl group and an optionally substituted carboxyl group;

a group represented by the following general formula (I)-4:

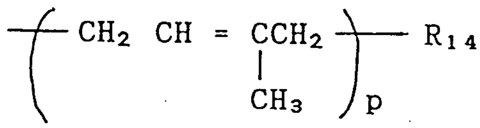

(I) - 4

wherein p is an integer of from 1 to 3; and

$R_{14}$ represents a hydrogen atom or an optionally branched alkyl group having 1 to 20 carbon atoms; and

a group represented by the following general formula (I)-5:

$- CH_2\ CH = CHR_{15}$ (I) - 5

wherein $R_{15}$ represents a hydrogen atom or a phenyl group; or

$R_6$ may form each of the groups represented by the following general formulae together with $R_5$:

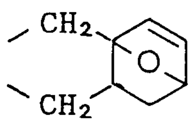

,

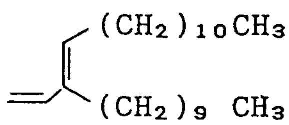

,

and

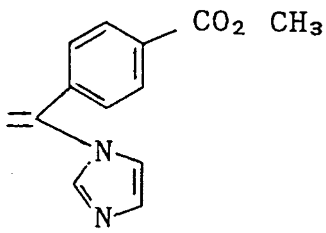

;

or a salt thereof;

a compound represented by the following general formula (II):

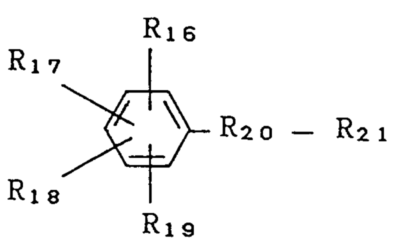

(II)

wherein $R_{16}$, $R_{17}$, $R_{18}$ and $R_{19}$ are each selected from a group consisting of a hydrogen atom, a hydroxy group, an optionally branched alkyl group having 1 to 5 carbon atoms and an alkoxy group having 1 to 5 carbon atoms;

$R_{20}$ is selected from a group consisting of O, S, a methylene group and a phenylene group; and

$R_{21}$ a group represented by the following general formula (II)-1:

$- NHR_{22}$ (II) - 1

wherein $R_{22}$ is selected from a group consisting of an optionally branched alkyl group having 1 to 15 carbon atoms, an optionally branched alkenyl group having 1 to 15 carbon atoms and a benzyl group;

and an optionally branched alkenyl group having 1 to 20 carbon atoms;
or a salt thereof;
a compound represented by the following general formula (III):

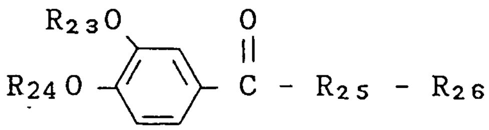

(III)

wherein $R_{23}$ and $R_{24}$ represent each a hydrogen atom or an acetyl group;
$R_{25}$ represents -NH- or a group represented by the following general formula:

$(CH2)_q$

wherein q is an integer of from 0 to 3;
$R_{26}$ is selected from a group consisting of a group represented by the following general formula (III)-1:

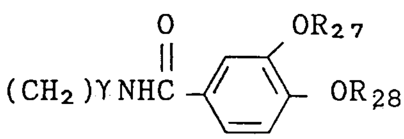

(III) - 1

wherein r is an integer of from 1 to 15; and
$R_{27}$ and $R_{28}$ represent each a hydrogen atom or an acetyl group;
a group represented by the following general formula (III)-2:

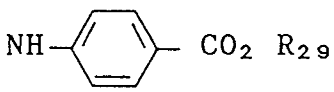

(III) - 2

wherein $R_{29}$ represents an alkyl group having 1 to 5 carbon atoms;
an optionally substituted phenyl group, an optionally substituted piperazinyl group and a pyridyl group;
or a salt thereof;
a compound represented by the following general formula (IV):

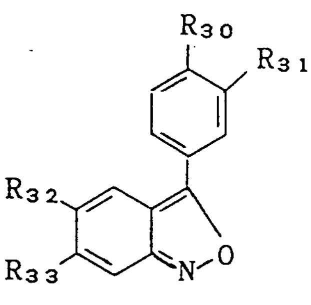

(IV)

wherein $R_{30}$ and $R_{31}$ represent each a hydrogen atom or a hydroxy group; and
$R_{32}$ and $R_{33}$ represent each a hydrogen atom or a halogen atom;
or a salt thereof;
a compound represented by the following general formula (V):

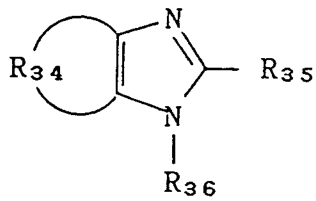

(V)

wherein $R_{34}$ forms a 5- to 7-membered ring which is optionally substituted and may contain 1 or 2 nitrogen atoms; and

$R_{35}$ and $R_{36}$ are each selected from a group consisting of a hydrogen atom, an optionally branched alkyl group having 1 to 20 carbon atoms and an optionally substituted alkenyl group having 1 to 20 carbon atoms;

or a salt thereof; and

a compound represented by the following general formula (VI):

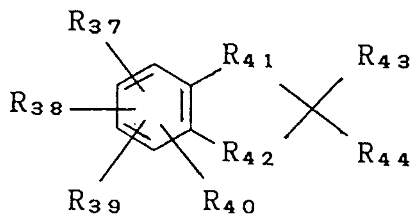

(VI)

wherein $R_{37}$, $R_{38}$, $R_{39}$ and $R_{40}$ are each selected from a group consisting of a hydrogen atom, a hydroxyl group and an alkoxy group having 1 to 5 carbon atoms;

$R_{41}$ is a group represented by the following general formula (VI)-1:

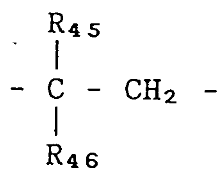

(VI) - 1

wherein $R_{45}$ and $R_{46}$ are each selected from a group consisting of a hydrogen atom, a hydroxy group and an alkyl group having 1 to 5 carbon atoms;

or each of the groups represented by the following general formulae:

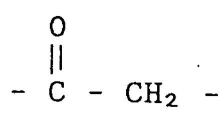

and

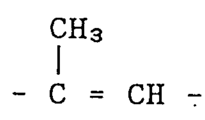

;

$R_{42}$ is an oxygen atom or a group represented by the following general formula (VI)-2:

$-NR_{47}-$ (VI) - 2

wherein $R_{47}$ is selected from a group consisting of a hydrogen atom, an alkyl group having 1 to 5 carbon atoms and a benzyl group; and

$R_{43}$ and $R_{44}$ are each selected from a group consisting of a hydrogen atom, an alkyl group having 1 to 5 carbon atoms and an optionally substituted phenyl group;

of a salt thereof.

**6.** A method for screening a remedy for arteriosclerosis which comprises examining an effect of

suppressing the negative charge of LDL by using agarose gel electrophoresis.

**Amended claims**

1. A drug composition which comprises a compound suppressing the negative charge of LDL and pharmaceutically acceptable carrier(s).

2. A drug composition as claimed in Claim 1, wherein the negative charge of LDL is confirmed by agarose gel electro-phoresis and/or the TBARS level due to the oxidation of LDL with $Cu^{2+}$.

3. A drug composition as claimed in Claim 1 or 2 which is a remedy for arteriosclerosis.

4. A drug composition as claimed in Claim 1 or 2 which is a treatment for peptic ulcers, cancer, ischemic organopathy, inflammation and pulmonary diseases caused by, for example, silicon dust.

5. A drug composition as claimed in each of Claims 1 to 4 which is a compound represented by the following general formulae (I) to (VI):
a compound represented by the general formula (I):

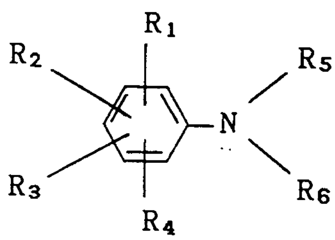

(I)

wherein $R_1$, $R_2$, $R_3$ and $R_4$ are each selected from a group consisting of a hydrogen atom, a hydroxy group, an optionally branched alkyl group having 1 to 5 carbon atoms, an alkoxy group having 1 to 5 carbon atoms, a methylthio group, a trimethylsilyloxy group, a methylenedioxy group, a halogen atom and a phenyl group;

$R_5$ is selected from a group consisting of a group represented by the following general formula (I)-1:

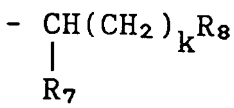

(I) - 1

wherein $R_7$ is selected from a group consisting of a hydrogen atom, an alkyl group having 1 to 5 carbon atoms, an alkenyl group having 1 to 5 carbon atoms, a phenyl group and a cyano group;

k is an integer of from 0 to 8; and

$R_8$ is selected from a group consisting of an optionally branched alkyl group having 1 to 20 carbon atoms, an optionally branched alkenyl group having 1 to 20 carbon atoms optionally substituted with a phenyl group, an optionally substituted phenyl group, an optionally substituted heterocyclic group, a cycloalkyl group having 3 to 8 carbon atoms, a naphthyl group, an adamantyl group, a tosyloxy group, a hydroxy group and a group represented by the following general formula:

$CO_2R_9$

wherein $R_9$ is selected from a group consisting of a hydrogen atom and an alkyl group having 1 to 5 carbon atoms;

a group represented by the following general formula (I)-2:

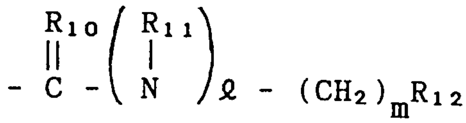 (I) - 2

wherein $R_{10}$ is selected from a group consisting of O, S and NCN;

$R_{11}$ represents a hydrogen atom or an optionally branched alkenyl group having 1 to 20 carbon atoms;

ℓ is an integer of 0 or 1;

m is an integer of from 0 to 10; and

$R_{12}$ is selected from a group consisting of an optionally branched alkyl group having 1 to 10 carbon atoms, an alkenyl group having 1 to 5 carbon atoms optionally substituted with a phenyl group, an alkoxy group having 1 to 5 carbon atoms, an optionally substituted phenyl group, a trifluoromethyl group, an alkylthio group having 1 to 20 carbon atoms, a halogen atom, a pyridyl group and a chloromethyl group;

a decalyl group, a tetralyl group, an adamantyl group, a tosyl group and a chromanyl group; and

$R_6$ is selected from a group consisting of a hydrogen atom, an alkyl group having 1 to 20 carbon atoms, a group represented by the following general formula (I)-3:

$-(CH_2)_n R_{13}$ (I) - 3

wherein n is an integer of from 1 to 6; and $R_{13}$ is selected from a group consisting of a hydroxy group, an optionally substituted phenyl group, a cyclohexyl group and an optionally substituted carboxyl group;

a group represented by the following general formula (I)-4:

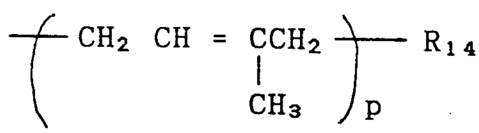 (I) - 4

wherein p is an integer of from 1 to 3; and

$R_{14}$ represents a hydrogen atom or an optionally branched alkyl group having 1 to 20 carbon atoms; and

a group represented by the following general formula (I)-5:

$-CH_2CH=CHR_{15}$ (I) - 5

wherein $R_{15}$ represents a hydrogen atom or a phenyl group; or

$R_6$ may form each of the groups represented by the following general formulae together with $R_5$:

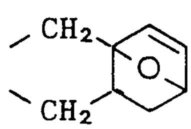 , 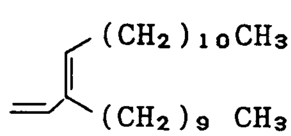 ,

and 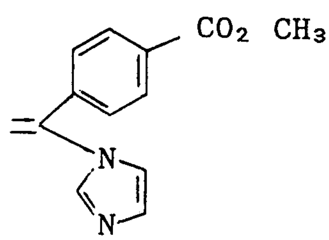 ;

or a salt thereof;

a compound represented by the following general formula (II):

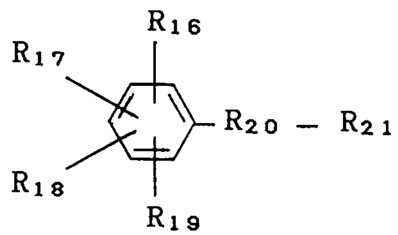

(II)

wherein $R_{16}$, $R_{17}$, $R_{18}$ and $R_{19}$ are each selected from a group consisting of a hydrogen atom, a hydroxy group, an optionally branched alkyl group having 1 to 5 carbon atoms and an alkoxy group having 1 to 5 carbon atoms;

$R_{20}$ is selected from a group consisting of O, S, a methylene group and a phenylene group; and

$R_{21}$ a group represented by the following general formula (II)-1:

$-NHR_{22}$ (II) - 1

wherein $R_{22}$ is selected from a group consisting of an optionally branched alkyl group having 1 to 15 carbon atoms, an optionally branched alkenyl group having 1 to 15 carbon atoms and a benzyl group;

and an optionally branched alkenyl group having 1 to 20 carbon atoms;

or a salt thereof;

a compound represented by the following general formula (III):

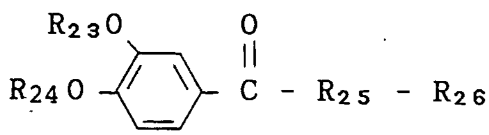

(III)

wherein $R_{23}$ and $R_{24}$ represent each a hydrogen atom or an acetyl group;

$R_{25}$ represents -NH- or a group represented by the following general formula:

$(CH2)q$

wherein q is an integer of from 0 to 3;

$R_{26}$ is selected from a group consisting of a group represented by the following general formula (III)-1:

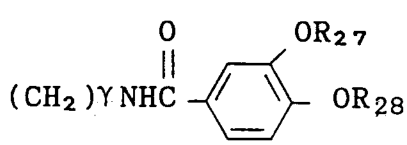

(III) - 1

wherein r is an integer of from 1 to 15; and

$R_{27}$ and $R_{28}$ represent each a hydrogen atom or an acetyl group;

a group represented by the following general formula (III)-2:

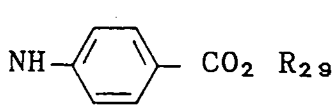

(III) - 2

wherein $R_{29}$ represents an alkyl group having 1 to 5 carbon atoms;

an optionally substituted phenyl group, an optionally substituted piperazinyl group and a pyridyl

group;
or a salt thereof;
a compound represented by the following general formula (IV):

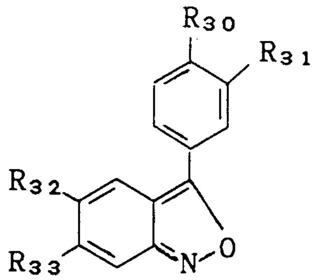

(IV)

wherein $R_{30}$ and $R_{31}$ represent each a hydrogen atom or a hydroxy group; and
$R_{32}$ and $R_{33}$ represent each a hydrogen atom or a halogen atom;
or a salt thereof;
a compound represented by the following general formula (V):

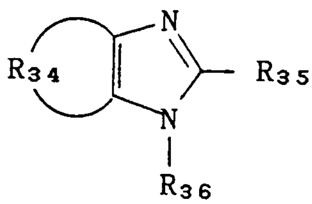

(V)

wherein $R_{34}$ forms a 5- to 7-membered ring which is optionally substituted and may contain 1 or 2 nitrogen atoms; and
$R_{35}$ and $R_{36}$ are each selected from a group consisting of a hydrogen atom, an optionally branched alkyl group having 1 to 20 carbon atoms and an optionally substituted alkenyl group having 1 to 20 carbon atoms;
or a salt thereof; and
a compound represented by the following general formula (VI):

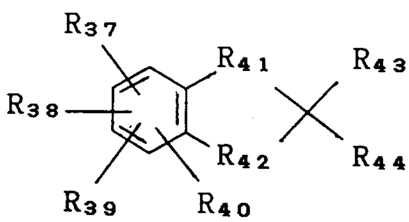

(VI)

wherein $R_{37}$, $R_{38}$, $R_{39}$ and $R_{40}$ are each selected from a group consisting of a hydrogen atom, a hydroxyl group and an alkoxy group having 1 to 5 carbon atoms; $R_{41}$ is a group represented by the following general formula (VI)-1:

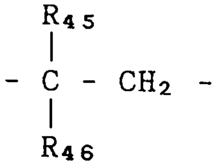

(VI) - 1

wherein $R_{45}$ and $R_{46}$ are each selected from a group consisting of a hydrogen atom, a hydroxy

group and an alkyl group having 1 to 5 carbon atoms;
or each of the groups represented by the following general formulae:

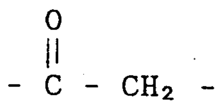 and 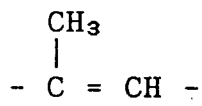 ;

$R_{42}$ is an oxygen atom or a group represented by the following general formula (VI)-2:

$-NR_{47}-$ (VI) - 2

wherein $R_{47}$ is selected from a group consisting of a hydrogen atom, an alkyl group having 1 to 5 carbon atoms and a benzyl group; and

$R_{43}$ and $R_{44}$ are each selected from a group consisting of a hydrogen atom, an alkyl group having 1 to 5 carbon atoms and an optionally substituted phenyl group;
of a salt thereof.

**6.** A method for screening a remedy for arteriosclerosis which comprises examining an effect of suppressing the negative charge of LDL by using agarose gel electrophoresis.

**7.** Process for the preparation of the drug composition according to claim 5 which comprises combining a compound represented by the general formulae (I) to (IV) as defined in claim 5 with a pharmaceutically acceptable carrier and/or diluent.

# INTERNATIONAL SEARCH REPORT

International Application No PCT/JP91/00179

**I. CLASSIFICATION OF SUBJECT MATTER** (if several classification symbols apply, indicate all) [6]

According to International Patent Classification (IPC) or to both National Classification and IPC

Int. $Cl^5$ A61K31/10, A61K31/12, A61K31/135, A61K31/155, A61K31/16, A61K31/17, A61K31/18, A61K31/185, A61K31/19

**II. FIELDS SEARCHED**

Minimum Documentation Searched [7]

| Classification System | Classification Symbols |
|---|---|
| IPC | A61K31/10, A61K31/12, A61K31/135, A61K31/155, A61K31/16, A61K31/17, A61K31/18, A61K31/185, A61K31/19 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 57-175119 (Chugai Pharmaceutical Co., Ltd.), October 28, 1982 (28. 10. 82), Claim & EP, A, 63383 | 1-5 |
| X | JP, A, 62-145049 (Mitsubishi Casei Corp.), June 29, 1987 (29. 06. 87), Claim & US, A, 4749701 & EP, A, 228959 | 1-5 |
| X | Chemical Abstracts, Vol.97, No.25, (1982), Abstract No. 216157g | 1-5 |
| X | Chemical Abstracts, Vol.108, No.5, (1988), Abstract No. 37726r | 1-5 |
| X | Chemical Abstracts, Vol.98, No.19, (1983), Abstract No. 160638r | 1-5 |
| X | Chemical Abstracts, Vol.91, No.3, (1979), Abstract No. 20144g | 1-5 |
| A | Chemical Abstracts, Vol.111, No.25, (1989), Abstract No. 229966c | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 8, 1991 (08. 05. 91) | May 20, 1991 (20. 05. 91) |
| **International Searching Authority** | **Signature of Authorized Officer** |
| Japanese Patent Office | |

International Application No. PCT/JP91/00179

**FURTHER INFORMATION CONTINUED FROM THE SECOND SHEET**

| | | |
|---|---|---|
| A | Chemical Abstracts, Vol.107, No.13, (1987), Abstract No. 113589v | 1-6 |
| A | Chemical Abstracts, Vol.106, No.7, (1987), Abstract No. 48497b | 1-6 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers ..... , because they are dependent claims and are not drafted in accordance with the second and third sentences of PCT Rule 6.4(a).

**VI.☒ OBSERVATIONS WHERE UNITY OF INVENTION IS LACKING [2]**

This International Searching Authority found multiple inventions in this international application as follows:

1.☒ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.